# EUROPÄISCHE PATENTSCHRIFT

(11) **EP 0 938 584 B1**
(45) Veröffentlichungstag und Bekanntmachung des Hinweises auf die Patenterteilung: **28.05.2008**
(21) Anmeldenummer: 97938817.0
(22) Anmeldetag: 10.07.1997
(51) Int. Cl.: C12P 41/00, C12N 9/80

(54) **VERFAHREN ZUR HERSTELLUNG VON (S)- ODER (R)-3,3,3-TRIFLUOR-2-HYDROXY-2-METHYLPROPIONSAÜRE**
METHOD OF PREPARING (S) - OR (R) -3,3,3-TRIFLUORO-2-HYDROXY-2- METHYLPROPIONIC ACID
PROCEDE DE PRODUCTION D'ACIDE (S)- OU (R)-3,3,3-TRIFLUORO-2-HYDROXY-2-METHYLPROPIONIQUE

(30) Priorität: 10.07.1996 CH 172396; 03.03.1997 CH 50097
(43) Veröffentlichungstag der Anmeldung: 01.09.1999
(73) Patentinhaber: Lonza AG, 3930 Visp (CH)
(72) Erfinder: BRIEDEN, Walter, CH-3902 Glis (CH); NAUGHTON, Andrew, CH-3930 Visp (CH); ROBINS, Karen, CH-3930 Visp (CH); SHAW, Nicholas, CH-3930 Visp (CH); TINSCHERT, Andreas, CH-3900 Brig (CH); ZIMMERMANN, Thomas, CH-3904 Naters (CH)
(74) Vertreter: Riegler, Norbert Hermann
(86) Internationale Anmeldenummer: PCT/EP1997/003670
(87) Internationale Veröffentlichungsnummer: WO 1998/001568

(56) Entgegenhaltungen:
- EP-A- 0 356 912
- EP-A- 0 433 117
- EP-A- 0 524 781

### SEQUENZPROTOKOLL

(1) ALLGEMEINE ANGABEN:
   (i) ANMELDER:
      (A) NAME: LONZA AG
      (B) STRASSE: Muenchensteinerstrasse 38
      (C) ORT: Basel
      (E) LAND: Schweiz
      (F) POSTLEITZAHL: 4002
   (ii) BEZEICHNUNG DER ERFINDUNG: Verfahren zur Herstellung von (S)- oder (R)-3,3,3-trifluor-2-hydroxy-2-mechylpropionsaeure
   (iii) ANZAHL DER SEQUENZEN: 14
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: Patent In Release #1.0, Version #1.30 (EPA)
(2) ANGABEN ZU SEQ ID NO: 1:
   (i) SEQUENZKENNZEICHEN:
      (A) LÄNGE: 1442 Basenpaare
      (B) ART: Nucleotid
      (C) STRANGFORM: Doppelstrang
      (D) TOPOLOGIE: ringförmig
   (ii) ART DES MOLEKÜLS: Genom-DNA
   (iii) HYPOTHETISCH: NEIN
   (iv) ANTISENSE: NEIN
   (vi) URSPRÜNLICHE HERKUNFT:
      (A) ORGANISMUS: Klebsiella oxytoca
      (B) STAMM: PRS1
      (C) INDIVIDUUM/ISOLAT: PRS1
   (vii) UNMITTELBARE HERKUNFT:
      (B) CLON(E): pPRS2a
   (ix) MERKMAL:
      (A) NAME/SCHLÜSSEL: CDS
      (B) LAGE:join(197..1181)
      (D) SONSTIGE ANGABEN:/product= "Amidase"
   (xi) SEQUENZBESCHREIBUNG: SEQ ID NO: 1:
   CCCGGGAACT CCATGTGGCC GTGATCCTGG TCGAGCAGGA TATTGCGATG ATCCAGCGGG      60
   CCGCACAGCG CTGTGCGGTA ATGGATAAAG GCCTGGTTGT AGAAACGCTG ACCCAACAAC     120
   AGCTCTCTGA TGATCTTTTA ATGCGTCGTC ATCTGGCTCT GTAACTAAAC GCTATAAATT     180
   ACGTGGAGAA TAACAT ATG AAA TGG TTG GAA GAA TCC ATT ATG GCC AAA         229
                     Met Lys Trp Leu Glu Glu Ser Ile Met Ala Lys
                      1               5                  10
   CGC GGT GTT GGT GCC GGG CGT AAA CCG GTA ACG CAT CAC CTG ACG GAA      277
   Arg Gly Val Gly Ala Gly Arg Lys Pro Val Thr His His Leu Thr Glu
               15                  20                  25
   GAA ATG CAA AAA GAG TTT CAT TAC ACC ATT GGC CCT TAT TCC ACA CCC      325
   Glu Met Gln Lys Glu Phe His Tyr Thr Ile Gly Pro Tyr Ser Thr Pro
           30                  35                  40
   GTC CTG ACC ATC GAA CCC GGT GAC CGG ATT ATT GTC GAC ACT CGA GAT      373
   Val Leu Thr Ile Glu Pro Gly Asp Arg Ile Ile Val Asp Thr Arg Asp
       45                  50                  55
   GCT TTT GAA GGT GCT ATC AAT TCG GAA CAG GAT ATT CCG AGC CAG TTG      421
   Ala Phe Glu Gly Ala Ile Asn Ser Glu Gln Asp Ile Pro Ser Gln Leu
   60                  65                  70                  75
   CTA AAA ATG CCC TTT CTC AAC CCA CAA AAC GGA CCG ATC ATG GTC AAT      469
   Leu Lys Met Pro Phe Leu Asn Pro Gln Asn Gly Pro Ile Met Val Asn
                   80                  85                  90
   GGC GCG GAG AAA GGT GAT GTG CTC GCT GTC TAT ATC GAA TCC ATG TTG      517
   Gly Ala Glu Lys Gly Asp Val Leu Ala Val Tyr Ile Glu Ser Met Leu
               95                  100                 105
   CCC CGC GGC GTT GAT CCC TAC GGC ATC TGC GCC ATG ATT CCG CAT TTT      565
   Pro Arg Gly Val Asp Pro Tyr Gly Ile Cys Ala Met Ile Pro His Phe
           110                 115                 120
   GGC GGA CTG ACC GGG ACC GAC CTG ACG GCC ATG CTC AAT GAT CCG CTG      613
   Gly Gly Leu Thr Gly Thr Asp Leu Thr Ala Met Leu Asn Asp Pro Leu
       125                 130                 135
   CCA GAA AAG GTG CGC ATG ATT AAA CTC GAC AGT GAA AAG GTC TAC TGG      661
   Pro Glu Lys Val Arg Met Ile Lys Leu Asp Ser Glu Lys Val Tyr Trp
   140                 145                 150                 155
   AGC AAA CGC CAT ACG CTT CCC TAT AAA CCC CAT ATT GGC ACC TTG AGC      709
   Ser Lys Arg His Thr Leu Pro Tyr Lys Pro His Ile Gly Thr Leu Ser
                   160                 165                 170
   GTA TCG CCA GAA ATT GAC TCA ATC AAT TCA CTG ACG CCA GAC AAT CAC      757
   Val Ser Pro Glu Ile Asp Ser Ile Asn Ser Leu Thr Pro Asp Asn His
               175                 180                 185
   GGC GGG AAT ATG GAT GTG CCG GAT ATA GGA CCA GGG AGT ATT ACC TAT      805
   Gly Gly Asn Met Asp Val Pro Asp Ile Gly Pro Gly Ser Ile Thr Tyr
           190                 195                 200
   CTG CCG GTA CGT GCG CCT GGA GGC CGC CTG TTT ATT GGT GAT GCC CAT      853
   Leu Pro Val Arg Ala Pro Gly Gly Arg Leu Phe Ile Gly Asp Ala His
       205                 210                 215
   GCT TGT CAG GGT GAT GGT GAG ATT TGC GGG ACC GCA GTA GAG TTT GCC      901
   Ala Cys Gln Gly Asp Gly Glu Ile Cys Gly Thr Ala Val Glu Phe Ala
   220                 225                 230                 235
   TCA ATC ACC ACC ATC AAA GTC GAT TTG ATC AAG AAC TGG CAG CTT TCC      949
   Ser Ile Thr Thr Ile Lys Val Asp Leu Ile Lys Asn Trp Gln Leu Ser
                   240                 245                 250
   TGG CCA CGA ATG GAG AAT GCC GAA AAT ATT ATG AGT ATT GGC AGT GCA      997
   Trp Pro Arg Met Glu Asn Ala Glu Asn Ile Met Ser Ile Gly Ser Ala
               255                 260                 265
   CGT CCG CTG GAG GAT GCG ACG CGA ATT GCA TAT CGC GAC TTA ATT TAC     1045
   Arg Pro Leu Glu Asp Ala Thr Arg Ile Ala Tyr Arg Asp Leu Ile Tyr
           270                 275                 280
   TGG CTG GTA GAA GAC TTT GGC TTC GAA CAA TGG GAT GCC TAC ATG CTT     1093
   Trp Leu Val Glu Asp Phe Gly Phe Glu Gln Trp Asp Ala Tyr Met Leu
       285                 290                 295
   CTG AGT CAA TGC GGC AAA GTG CGG CTG GGC AAC ATG GTC GAC CCC AAA     1141
   Leu Ser Gln Cys Gly Lys Val Arg Leu Gly Asn Met Val Asp Pro Lys
   300                 305                 310                 315
   TAC ACC GTT GGC GCG ATG CTG AAC AAA AAC CTG TTA GTT TAGTAGGAAT      1190
   Tyr Thr Val Gly Ala Met Leu Asn Lys Asn Leu Leu Val
                   320                 325
   AACTAACCGG TGAACATTAC CCGGATGTAG ATCGGGGTAA TGTGTAAGTT CAAACAATCG   1250
   CTATTTTTAA CAGCTAAAGC AGGTGCATAT GGGGCCAGAT ACACCCATCA ATATTGGTTT   1310
   ACTTTACTCC TTCAGCGGAG TGACGGCGGC ACAAGAGTTG TCACAATGGC GCGGAGCAAC   1370
   CCAGGCTATT GCCGAAATTA ATCAAAATGG CGGCATCAAC GGCAGACCAC TCAATGCAAT   1430
   TCATTTGGAT CC                                                       1442
(2) ANGABEN ZU SEQ ID NO: 2:
   (i) SEQUENZKENNZEICHEN:
      (A) LÄNGE: 328 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 2:
   Met Lys Trp Leu Glu Glu Ser Ile Met Ala Lys Arg Gly Val Gly Ala
   1               5                   10                  15
   Gly Arg Lys Pro Val Thr His His Leu Thr Glu Glu Met Gln Lys Glu
               20                  25                  30
   Phe His Tyr Thr Ile Gly Pro Tyr Ser Thr Pro Val Leu Thr Ile Glu
           35                  40                  45
   Pro Gly Asp Arg Ile Ile Val Asp Thr Arg Asp Ala Phe Glu Gly Ala
       50                  55                  60
   Ile Asn Ser Glu Gln Asp Ile Pro Ser Gln Leu Leu Lys Met Pro Phe
   65                  70                  75                  80
   Leu Asn Pro Gln Asn Gly Pro Ile Met Val Asn Gly Ala Glu Lys Gly
                   85                  90                  95
   Asp Val Leu Ala Val Tyr Ile Glu Ser Met Leu Pro Arg Gly Val Asp
               100                 105                 110
   Pro Tyr Gly Ile Cys Ala Met Ile Pro His Phe Gly Gly Leu Thr Gly
           115                 120                 125
   Thr Asp Leu Thr Ala Met Leu Asn Asp Pro Leu Pro Glu Lys Val Arg
       130                 135                 140
   Met Ile Lys Leu Asp Ser Glu Lys Val Tyr Trp Ser Lys Arg His Thr
   145 150 155 160
   Leu Pro Tyr Lys Pro His Ile Gly Thr Leu Ser Val Ser Pro Glu Ile
   165 170 175
   Asp Ser Ile Asn Ser Leu Thr Pro Asp Asn His Gly Gly Asn Met Asp
   180 185 190
   Val Pro Asp Ile Gly Pro Gly Ser Ile Thr Tyr Leu Pro Val Arg Ala
   195 200 205
   Pro Gly Gly Arg Leu Phe Ile Gly Asp Ala His Ala Cys Gln Gly Asp
   210 215 220
   Gly Glu Ile Cys Gly Thr Ala Val Glu Phe Ala Ser Ile Thr Thr Ile
   225 230 235 240
   Lys Val Asp Leu Ile Lys Asn Trp Gln Leu Ser Trp Pro Arg Met Glu
   245 250 255
   Asn Ala Glu Asn Ile Met Ser Ile Gly Ser Ala Arg Pro Leu Glu Asp
   260 265 270
   Ala Thr Arg Ile Ala Tyr Arg Asp Leu Ile Tyr Trp Leu Val Glu Asp
   275 280 285
   Phe Gly Phe Glu Gln Trp Asp Ala Tyr Met Leu Leu Ser Gln Cys Gly
   290 295 300
   Lys Val Arg Leu Gly Asn Met Val Asp Pro Lys Tyr Thr Val Gly Ala
   305 310 315 320
   Met Leu Asn Lys Asn Leu Leu Val
   325
(2) ANGABEN ZU SEQ ID NO: 3:
   (i) SEQUENZKENNZEICHEN:
      (A) LÄNGE: 20 Aminosäuren
      (B) ART: Aminosäure
      (C) STRANGFORM: nicht bekannt
      (D) TOPOLOGIE: nicht bekannt
   (ii) ART DES MOLEKÜLS: Peptid
   (vi) URSPRÜNLICHE HERKUNFT:
      (B) STAMM: PRS1
      (C) INDIVIDUUM/ISOLAT: PRS1
   (xi) SEQUENZBESCHREIBUNG: SEQ ID NO: 3:
   Met Lys Trp Leu Glu Glu Ser Ile Met Ala Lys Arg Gly Val Gly Ala
   1 5 10 15
   Ser Arg Lys Pro
   20
(2) ANGABEN ZU SEQ ID NO: 4:
   (i) SEQUENZKENNZEICHEN:
      (A) LÄNGE: 5 Aminosäuren
      (B) ART: Aminosäure
      (C) STRANGFORM: nicht bekannt
      (D) TOPOLOGIE: nicht bekannt
   (ii) ART DES MOLEKÜLS: Peptid
   (vi) URSPRÜNLICHE HERKUNFT:
      (B) STAMM: PRS1
      (C) INDIVIDUUM/ISOLAT: PRS1
   (xi) SEQUENZBESCHREIBUNG: SEQ ID NO: 4:
   Val Tyr Trp Ser Lys
   1               5
(2) ANGABEN ZU SEQ ID NO: 5:
   (i) SEQUENZKENNZEICHEN:
      (A) LÄNGE: 13 Aminosäuren
      (B) ART: Aminosäure
      (C) STRANGFORM: nicht bekannt
      (D) TOPOLOGIE: nicht bekannt
   (ii) ART DES MOLEKÜLS: Peptid
   (vi) URSPRÜNLICHE HERKUNFT:
      (B) STAMM: PRS1
      (C) INDIVIDUUM/ISOLAT: PRS1
   (xi) SEQUENZBESCHREIBUNG: SEQ ID NO: 5:
   Lys Pro Val Thr His His Leu Thr Glu Glu Met Gln Lys
   1               5                   10
(2) ANGABEN ZU SEQ ID NO: 6:
   (i) SEQUENZKENNZEICHEN:
      (A) LÄNGE: 9 Aminosäuren
      (B) ART: Aminosäure
      (C) STRANGFORM: nicht bekannt
      (D) TOPOLOGIE: nicht bekannt
   (ii) ART DES MOLEKÜLS: Peptid
   (vi) URSPRÜNLICHE HERKUNFT:
      (B) STAMM: PRS1
      (C) INDIVIDUUM/ISOLAT: PRS1
   (xi) SEQUENZBESCHREIBUNG: SEQ ID NO: 6:
   Tyr Thr Val Gly Ala Met Leu Asn Lys
   1               5
(2) ANGABEN ZU SEQ ID NO: 7:
   (i) SEQUENZKENNZEICHEN:
      (A) LÄNGE: 14 Aminosäuren
      (B) ART: Aminosäure
      (C) STRANGFORM: nicht bekannt
      (D) TOPOLOGIE: nicht bekannt
   (ii) ART DES MOLEKÜLS: Peptid
   (vi) URSPRÜNLICHE HERKUNFT:
      (B) STAMM: PRS1
      (C) INDIVIDUUM/ISOLAT: PRS1
   (xi) SEQUENZBESCHREIBUNG: SEQ ID NO: 7:
   Met Glu Asn Ala Glu Asn Ile Met Ser Ile Gly Ser Ala Arg
   1               5                   10
(2) ANGABEN ZU SEQ ID NO: 8:
   (i) SEQUENZKENNZEICHEN:
      (A) LÄNGE: 9 Aminosäuren
      (B) ART: Aminosäure
      (C) STRANGFORM: nicht bekannt
      (D) TOPOLOGIE: nicht bekannt
   (ii) ART DES MOLEKÜLS: Peptid
   (vi) URSPRÜNLICHE HERKUNFT:
      (B) STAMM: PRS1
      (C) INDIVIDUUM/ISOLAT: PRS1
   (xi) SEQUENZBESCHREIBUNG: SEQ ID NO: 8:
   Trp Leu Glu Glu Ser Ile Met Ala Lys
   1               5
(2) ANGABEN ZU SEQ ID NO: 9:
   (i) SEQUENZKENNZEICHEN:
      (A) LÄNGE: 18 Aminosäuren
      (B) ART: Aminosäure
      (C) STRANGFORM: nicht bekannt
      (D) TOPOLOGIE: nicht bekannt
   (ii) ART DES MOLEKÜLS: Peptid
   (vi) URSPRÜNLICHE HERKUNFT:
      (B) STAMM: PRS1
      (C) INDIVIDUUM/ISOLAT: PRS1
   (xi) SEQUENZBESCHREIBUNG: SEQ ID NO: 9:
   Met Pro Phe Leu Asn Pro Gln Asn Gly Pro Ile Met Val Asn Gly Ala
   1               5                   10                  15
   Glu Lys
(2) ANGABEN ZU SEQ ID NO: 10:
   (i) SEQUENZKENNZEICHEN:
      (A) LÄNGE: 19 Aminosäuren
      (B) ART: Aminosäure
      (C) STRANGFORM: nicht bekannt
      (D) TOPOLOGIE: nicht bekannt
   (ii) ART DES MOLEKÜLS: Peptid
   (vi) URSPRÜNLICHE HERKUNFT:
      (B) STAMM: PRS1
      (C) INDIVIDUUM/ISOLAT: PRS1
   (xi) SEQUENZBESCHREIBUNG: SEQ ID NO: 10:
   Asp Ala Phe Glu Gly Ala Ile Asn Ser Glu Gln Asp Ile Pro Ser Gln
   1               5                   10                  15
   Leu Leu Lys
(2) ANGABEN ZU SEQ ID NO: 11:
   (i) SEQUENZKENNZEICHEN:
      (A) LÄNGE: 21 Aminosäuren
      (B) ART: Aminosäure
      (C) STRANGFORM: nicht bekannt
      (D) TOPOLOGIE: nicht bekannt
   (ii) ART DES MOLEKÜLS: Peptid
   (vi) URSPRÜNLICHE HERKUNFT:
      (B) STAMM: PRS1
      (C) INDIVIDUUM/ISOLAT: PRS1
   (xi) SEQUENZBESCHREIBUNG: SEQ ID NO: 11:
   Glu Phe His Tyr Thr Ile Gly Pro Tyr Ser Thr Pro Val Leu Thr Ile
   1               5                   10                  15
   Glu Pro Gly Asp Arg
               20
(2) ANGABEN ZU SEQ ID NO: 12:
   (i) SEQUENZKENNZEICHEN:
      (A) LÄNGE: 23 Aminosäuren
      (3) ART: Aminosäure
      (C) STRANGFORM: nicht bekannt
      (D) TOPOLOGIE: nicht bekannt
   (ii) ART DES MOLEKÜLS: Peptid
   (vi) URSPRÜNLICHE HERKUNFT:
      (B) STAMM: PRS1
      (C) INDIVIDUUM/ISOLAT: PRS1
   (xi) SEQUENZBESCHREIBUNG: SEQ ID NO: 12:
   Leu Phe Ile Gly Asp Ala His Ala Glu Gln Gly Asp Gly Glu Ile Glu
   1               5                   10                  15
   Gly Thr Ala Val Glu Phe Ala
               20
(2) ANGABEN ZU SEQ ID NO: 13:
   (i) SEQUENZKENNZEICHEN:
      (A) LÄNGE: 14 Aminosäuren
      (B) ART: Aminosäure
      (C) STRANGFORM: nicht bekannt
      (D) TOPOLOGIE: nicht bekannt
   (ii) ART DES MOLEKÜLS: Peptid
   (vi) URSPRÜNLICHE HERKUNFT:
      (B) STAMM: PRS1
      (C) INDIVIDUUM/ISOLAT: PRS1
   (xi) SEQUENZBESCHREIBUNG: SEQ ID NO: 13:
   Gly Asp Val Leu Ala Val Tyr Ile Glu Ser Met Leu Pro Arg
   1               5                   10
(2) ANGABEN ZU SEQ ID NO: 14:
   (i) SEQUENZKENNZEICHEN:
      (A) LÄNGE: 33 Aminosäuren
      (B) ART: Aminosäure
      (C) STRANGFORM: nicht bekannt
      (D) TOPOLOGIE: nicht bekannt
   (ii) ART DES MOLEKÜLS: Peptid
   (vi) URSPRÜNLICHE HERKUNFT:
      (C) INDIVIDUUM/ISOLAT: PRS1
   (vii) UNMITTELBARE HERKUNFT:
      (B) CLON(E): PRS1
   (xi) SEQUENZBESCHREIBUNG: SEQ ID NO: 14:
   Gly Val Asp Pro Tyr Gly Ile Glu Ala Met Ile Pro His Phe Gly Gly
   1               5                   10                  15
   Leu Thr Gly Thr Asp Leu Thr Ala Met Leu Asn Asp Gln Leu Gln Pro
               20                  25                  30
   Lys

## Beschreibung

Die vorliegende Erfindung betrifft ein neues Verfahren zur Herstellung von (S)- oder (R)-3,3,3-Trifluor-2-hydroxy-2-methylpropionsaure sowie neue Mikroorganismen, die befähigt sind das Propionsäureamid der Formel 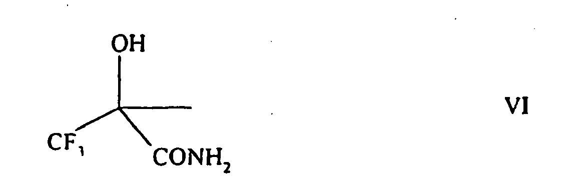 in Form des Racemats oder seiner optisch aktiven Isomere als einzige Stickstoffquelle zu verwerten
(S)-3,3,3-Trifluor-2-hydroxy-2-methylpropionsaure ist ein wichtiges Zwischenprodukt zur Herstellung von therapeutischen Amiden (EP-A 0 524 781).

Im folgenden wird 3,3,3-Trifluor-2-hydroxy-2-methylpropionsäure mit 2,2-HTFMPS und 3,3,3-Trifluor-2-hydroxy-2-methylpropionsäureamid mit 2,2-HTFMPA abgekürzt

Gemäss J. Chem Soc. 1951. S 2329 wird ein Verfahren zur Herstellung von (S)-2,2-HTFMPS beschrieben, bei dem das entsprechende Racemat mittels Dimethoxystrychnin in das gewunschte (S)-Enantiomere uberfuhrt wird. Dieses Verfahren hat den Nachteil, dass das fur die Racemattrennung eingesetzte Dimethoxystrychnin zu kostspielig ist

Die EP-A 0 524 781 beschreibt ein Verfahren zur Herstellung von (S)-HTFMPS bei dem das entsprechende Racemat mittels (S)-(-)-α-Methylbenzylamin in das gewünschte (S)-Enantiomere überfuhrt wird Nachteilig bei diesem Verfahren ist, dass grosse Mengen an (S)-(-)-α-Methylbenzylamin eingesetzt werden müssen, womit dieses Verfahren ebenfalls zu kostspielig ist

Aufgabe der vorliegenden Erfindung ist es, ein kostengünstiges und technisch gangbares Verfahren zur Herstellung von (S)- oder (R)-2,2-HTFMPS zur Verfugung zu stellen

Diese Aufgabe wird mit den erfindungsgemässen Mikroorganismen gemäss Anspruch 1 und Anspruch 11, den Polypeptiden gemass Anspruch 4 und mit den Verfahren gemass den Ansprüchen 15 und 16 gelöst.

Gegenstand der vorliegenden Erfindung sind demnach aus der Natur selektionierte Mikroorganismen, sogenannte "Wildstämme", Enzymextrakte davon, die aus ihnen isolierten Enzyme mit stereospezifischer Amidohydrolase-Aktivitat sowie die aus den "Wildstämmen" isolierte(n) DNA / DNA-Fragmente, die für eine sterospezifische Amidohydrolase codieren. Gegenstand der vorliegenden Erfindung sind ferner sogenannte gentechnologisch veränderte Mikroorganismen, die diese DNA-Fragmente bzw. Vektoren enthalten. Ein weiterer Gegenstand ist ein Verfahren zur Herstellung von (S)- oder (R)-2,2-HTFMPS und ein Verfahren zur Herstellung von (S)- oder (R)-2,2-HTFMPA unter Verwendung der beschriebenen Mikroorganismen

Die Erfindung wird durch die nachfolgenden Abbildungen näher erläutert
- Fig. 1: zeigt die Restriktionskarte der isolierten DNA
- Fig 2: zeigt Plasmid pPRS1b
- Fig. 3: zeigt Plasmid pPRS2a
- Fig. 4: zeigt das pH-Optimum der Amidohydrolase
- Fig. 5: zeigt die Michaelis-Menten-Kinetik der Amidohydrolase
- Fig 6: zeigt das Temperaturoptimum der Amidohydrolase
- Fig. 7: zeigt den Effekt von Methanol der Amidohydrolase

Die erfindungsgemassen "Wildstamme" können aus Bodenproben, Schlamm oder Abwasser unter Zuhilfenahme ublicher mikrobiologischer Techniken isoliert werden.
Erfindungsgemass erfolgt die Isolation derart, dass man diese in einem Medium enthaltend das Propionsäureamid der Formel VI in Form des Racemats oder eines seiner optisch aktiven Isomere als einzige Stickstoffquelle mit einer geeigneten Kohlenstoffquelle auf übliche Weise züchtet. Aus der durch Züchtung erhaltenen Kultur werden dann jene selektioniert, die stabil sind und das Propionsäureamid der Formel VI als einzige Stickstoffquelle verwerten.

Als geeignete Kohlenstoffquellen konnen die "Wildstamme" Zucker, Zuckeralkohole oder Carbonsauren als Wachstumssubstrat nutzen. Als Zucker können z. B Glucose, Arabinose, Rhamnose, Lactose oder Maltose verwendet werden. Als Zuckeralkohole können bspw. Sorbit, Mannit oder Glycerin verwendet werden. Als Carbonsäure kann beispielsweise Citronensäure verwendet werden. Vorzugsweise wird als Kohlenstoffquelle Glycerin oder Glucose eingesetzt.

Als-Selektions- und Anzuchtmedium können die in der Fachwelt üblichen verwendet werden, wie beispielsweise ein Mineralsalzmedium gemäss Kulla et al., Arch. Microbiol. 135, S. 1 - 7, 1983.

Während der Anzucht und Selektion werden zweckmässig die wirksamen Enzyme der Mikroorganismen induziert. Als Enzyminduktor kann das Propionsäureamid der Formel VI in Form des Racemats oder eines seiner optisch aktiven Isomere, Acetamid oder Malonsäurediamid, verwendet werden.

Üblicherweise erfolgt die Anzucht und Selektion bei einer Temperatur von 0 bis 42 °C, vorzugsweise von 20 bis 37 °C und bei einem pH-Wert von 4 bis 9, vorzugsweise bei einem pH-Wert von 6 bis 8.

Bevorzugte "Wildstämme" sind Propionsäureamid (Formel VI) verwertende der Gattung Klebsiella. Ganz besonders bevorzugt sind Mikroorganismen der Spezies Klebsiella oxytoca PRS 1 (DSM 11009), Klebsiella oxytoca PRS 1K17 (DSM 11623) Pseudomonas sp. (DSM 11010), Rhodococcus opacus ID-622 (DSW 11344), Arthrobacter ramosus ID-620 (DSM 11350), Bacillus sp. ID-621 (DSM 11351), Klebsiella planticula ID-624 (DSM 11354) und Klebsiella pneumoniae ID-625 (DSM 11355), sowie deren funktionelle aequivalente Varianten und Mutanten. Dabei weisen die "Wildstämme" Klebsiella oxytoca (DSM 11009), Klebsiella planticula ID-624 (DSM 11354) und Klebsiella pneumoniae ID-625 (DSM 11355) bevorzugt (R)-Amidohydrolase-Aktivität und die "Wildstämme" Pseudomonas sp. (DSM 11010), Rhodococcus opacus ID-622 (DSM 11344), Arthrobacter ramosus ID-620 (DSM 11350) und Bacillus sp. ID-621 (DSM 11351) bevorzugt (S)-Amidohydrolase-Aktivität auf. Die Mikroorganismen mit der Bezeichnung DSM 11010, DSM 11009 wurden am 24.06.1996, die Mikroorganismen mit der Bezeichnung DSM 11355, DSM 11354 am 27.12.1996, die Mikroorganismen mit der Bezeichnung DSM 11351, DSM 11350 und DSM 11344 am 13.12.1996 und die Mikroorganismen mit der Bezeichnung DSM 11623 am 20.06.1.997 bei der Deutschen Sammlung von Mikroorganismen und Zellkulturen GmbH, Mascheroderweg 1b, D-38124 Braunschweig, gemäss Budapester Vertrag hinterlegt.

Unter "funktionell äquivalenten Varianten und Mutanten" der "Wildstämme" werden Stämme verstanden, die im wesentlichen dieselben Eigenschaften und Funktionen wie die Ursprungsmikroorganismen besitzen. Derartige Varianten und Mutanten können zufällig z. B. durch UV-Bestrahlung gebildet werden oder gezielt durch chemische Mutagenese wie z. B. durch Interkalatoren, wie Acridin-Farbstoffe.

### Taxonomische Beschreibung von Klebsiella oxytoca PRS1 (DSM 11009)

| Zellform | Stäbchen |
|---|---|
| Breite µm | 1,0 - 1,2 |
| Länge µm | 1,2 - 2,0 |
| | |
| Beweglichkeit | - |
| | |
| Gram-Reaktion | - |
| Lyse durch 3% KOH | + |
| Aminopeptidase (Cerny) | + |
| | |
| Sporen | - |
| | |
| Oxidase | - |
| | |
| Catalase | + |
| | |
| Wachstum | |
| anaerob | + |
| | |
| Gas aus Glucose | + |
| | |
| Säure aus (ASS) | |
| Glucose | + |
| Fructose | + |
| Xylose | + |
| Erythrit | - |
| Adonit | + |
| D-Mannose | + |
| L-Rhamnose | + |
| Inosit | + |
| Sorbit | + |
| α-Methyl-D-glucosid | + |
| Cellobiose | + |
| Maltose | + |
| Lactose | + |
| D-Arabitol | + |
| | |
| ONPG | + |
| | |
| ADH | - |
| LDC | w |
| | |
| ODC | - |
| | |
| VP | + |
| | |
| Indol | + |
| | |
| H₂S-Bildung | - |
| | |
| Simmons Citrat | + |
| | |
| Urease | + |
| | |
| Methylrot | - |
| | |
| Hydrolyse von | |
| Gelatine | - |
| DNA | - |
| Tween 80 | - |

### Taxonomische Beschreibung von Pseudomonas sp. (DSM 11010)

| Zellform | Stäbchen | |
|---|---|---|
| Breite µm | | 0,7 - 0,8 |
| Länge µm | | 1,5 - 3,5 |
| | | |
| Beweglichkeit | + | |
| | | |
| Gram-Reaktion | - | |
| Lyse durch 3% KOH | + | |
| Aminopeptidase (Cerny) | + | |
| | | |
| Sporen | - | |
| | | |
| Oxidase | + | |
| | | |
| Fluoreszens | + | |
| | | |
| Catalase | + | |
| | | |
| Wachstum bei 41 °C | - | |
| | | |
| ADH | + | |
| | | |
| Urease | - | |
| | | |
| Hydrolyse von Gelatine | + | |
| | | |
| Nitratreduktion | - | |
| | | |
| Denitrifikation | - | |
| | | |
| Levan aus Saccharose | + | |
| | | |
| Lecithinase | + | |
| | | |
| Substratverwertung | | |
| Adipat | - | |
| Citrat | + | |
| Malat | + | **Abkürzungen**: |
| L-Mandelat | - | **ASS** Acetylsalicylsäure |
| Phenylacetat | - | **ONPG** O-Nitro-phenylgalactosidase |
| D-Glucose | + | **ADH:** Alkoholdehydrogenase |
| Maltose | - | **LDC** Lactatdecarboxylase |
| Trehalose | + | **ODC** Ornithindecarboxylase |
| Mannitol | + | **VP** Voges Proskauer |
| Adonitol | + | |
| Acetamid | + | |
| Hippurat | - | |
| Tryptamin | - | |
| Butylamin | - | |

Das erfindungsgemasse Enzym mit stereospezifischer Amidohydrolase-Aktivität ist beispielsweise aus den bereits beschriebenen "Wildstammen" erhaltlich und befähigt, das Propionsäureamid der Formel 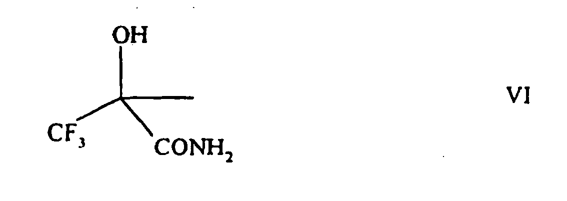 in Form des Racemats oder seines (R)-Isomeren zu hydrolysieren, sowie funktionell aequivalente Varianten und Mutanten davon

Unter "funktionell aequivalenten Varianten und Mutanten" der Enzyme werden Enzyme verstanden, die im wesentlichen dieselben Eigenschaften und Funktionen besitzen Derartige Varianten und Mutanten können zufällig z. B. durch Mutation gebildet werden

Zweckmässig ist das Enzym charakterisiert durch
a) ein pH-Optimum von pH 10 ± 0,5
b) ein Temperaturoptimum zwischen 65 und 70 °C bei einem pH-Wert von 10 und
c) einem K_{M}-Wert fur das Substrat (R)-2,2-HTFMPA von 32 mM (60 °C in 100 mM CAPS-Puffer (3-(Cyclohexylamino)-1-propansulfonsaure) pH 10),
   insbesondere dadurch, dass
d) eine 5 bis 20% Methanolkonzentration inhibierend wirkt und
e) die N-terminale Aminosäuresequenz: Met-Lys-Trp-Leu-Glu-Glu-Ser-Ile-Met-Ala-Lys-Arg-Gly-Val-Gly-Ala-Ser-Arg-Lys-Pro ist.

Diese stereospezifische Amidohydrolase kann aus den bereits beschriebenen "Wildstammen" isoliert werden, die befähigt sind, das Propionsäureamid der Formel VI in Form des Racemats oder seines R-Isomeren als einzige Stickstoffquelle zu verwerten Zweckmässig wird die Amidohydrolase aus den "Wildstämmen" der Gattung Klebsiella, bevorzugt aus Klebsiella oxytoca PRS 1 (DSM 11009) oder Klebsiella oxytoca PRS1K17 (DSM 11623) isoliert.

Selbstverständlich kann dieses Enzym ebenso aus den von diesen "Wildstammen" abgeleiteten gentechnologisch veränderten Mikroorganismen isoliert werden

Zur Gewinnung der stereospezifischen Amidohydrolase werden die "Wildstamme" in einem wässrigen Nährmedium, das eine Kohlenstoff-, Stickstoffquelle, Mineralsalze und eine Vitaminquelle enthält, auf ubliche Weise gezuchtet (kultiviert). Zweckmässig werden die "Wildstämme" bei einer Temperatur von 20 bis 35 °C und bei einem pH-Wert von 6 bis 8 kultiviert. Das Enzym kann dann nach Aufschluss der Zellen z B. durch die French-Press durch an sich bekannte Methoden der Enzymreinigung isoliert werden

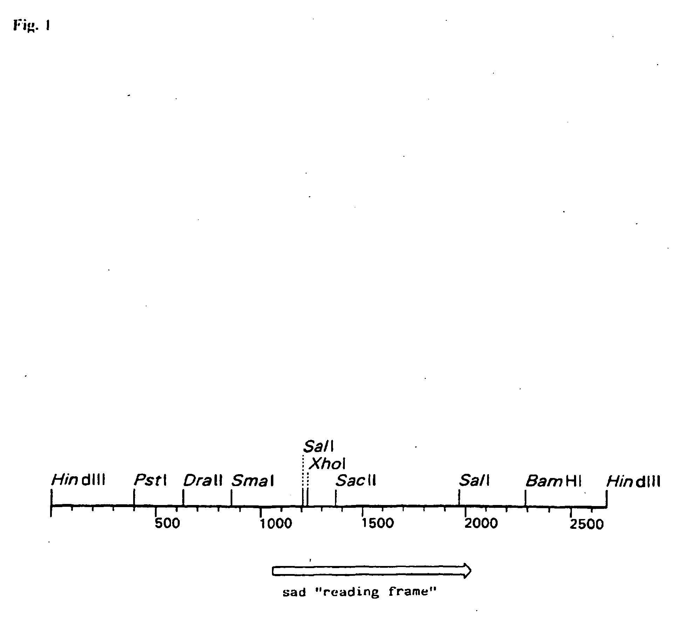

Die erfindungsgemasse DNA bzw. die erfindungsgemässen DNA-Fragmente, die fur eine stereospezifische Amidohydrolase codieren, wie sie insbesondere durch die Aminosäuresequenz in SEQ ID No 2 dargestellt wird, und die durch die Restriktionskarte gemäss Fig. 1 und insbesondere durch die Nukleotidsequenz in SEQ ID No. 1 charakterisiert sind, umfassen auch deren funktionell aequivalente genetische Varianten und Mutanten, d h Gene, die sich von den Genen der Wildtyp-Organismen ableiten und deren Genprodukte in ihrer biologischen Funktion im wesentlichen unverandert sind Die funktionell aequivalenten genetischen Varianten und Mutanten umfassen somit beispielsweise Basenaustausche im Rahmen der bekannten Degeneration des genetischen Codes, wie sie z. B. künstlich erzeugt werden können, um die Gensequenz an die bevorzugte Codon-Verwendung eines bestimmten Mikroorganismus, in dem eine Expression erfolgen soll, anzupassen Die genetischen Varianten und Mutanten umfassen auch Deletionen, Insertionen und Substitutionen von Basen oder Codons, soweit die Genprodukte derart veränderter Gene in ihrer biologischen Funktion im wesentlichen unverändert lassen. Umfasst werden hierdurch z. B. Gensequenzen, die zu den Wildtypsequenzen eine hohe Homologie, beispielsweise höher als 70% aufweisen und unter stringenten Hybridisierungsbedingungen, z B bei Temperaturen zwischen 60 und 70°C und bei 0,5 bis 1,5 M Salzanteil, insbesondere bei einer Temperatur von 67 °C und bei 0,8 M Salzanteil mit dem Komplement der Wildtypsequenzen zur Hybridisierung in der Lage sind.

Als Ausgangsmaterial fur die erfindungsgemässe DNA können die bereits beschriebenen "Wildstämme" dienen, die als Ausgangsmaterial zur Isolation der erfindungsgemässen stereospezifischen Amidohydrolase eingesetzt werden.

Die Isolierung der intakten Gene bzw der intakten erfindungsgemässen DNA-Fragmente kann nach bekannten Methoden ausgehend von einer Genbank eines geeigneten Mikroorganismus wie Klebsiella oxytoca erfolgen, aus der das Amidohydrolase-Gen, oder Fragmente davon durch Hybridisierung mit markierten Oligonukleotiden, die Teilsequenzen der Amidohydrolase-Gene enthalten, in bekannter Weise isoliert und kloniert werden konnen. Im folgenden wird das Amidohydrolase-Gen als sad abgekürzt,

Zur Verbesserung der Transkription wird das sad-Gen zweckmassig unter die Kontrolle eines starken Promotors gestellt Die Wahl des Promotors hängt von den gewünschten Expressionsbedingungen ab, beispielsweise davon, ob eine konstitutive oder induzierte Expression gewunscht wird, oder von dem Mikroorganismus, in dem die Expression erfolgen soll

Geeignete Promotoren sind die Promotoren P_{L} und P_{R} des Phagen Lambda (vgl. Schauder et al.., Gene. 52, 279 - 283, 1987), der P_{trc}-Promotor (Amann et al , Gene, 69, 301 - 315. 1988), die Promotoren P_{Nm}, P_{SI} (M Labes et al., Gene, 89, 37 - 46, 1990), der Pₜᵣₚ-Promotor (Amann et al, Gene, 25. 167 - 178, 1983), der P_{lac}-Promotor (Amann et al. Gene, 25 167 - 178, 1983) und der P_{tac}-Promotor, ein Hybrid aus den genannten Pₜᵣₚ- und P_{lac}-Promotoren, der als konstitutiver oder induzierbarer Promotor eingesetzt werden kann (Russel und Bennett. Gene, 20, 231 - 243, 1982). Bevorzugt wird der P_{lac}-Promotor verwendet

Zur Verwendung bei der Produktion von z B (R)-2,2-HTFMPS in einem geeigneten Produktionsstamm werden die erfindungsgemassen DNA-Fragmente zweckmässig mit Hilfe bekannter Techniken in bekannte geeignete Vektoren, vorzugsweise Expressionsvektoren eingebaut
Als Vektoren konnen autonom und selbstreplizierende Plasmide oder Integrationsvektoren verwendet werden

Abhängig von der Art der gewählten Vektoren können die sad-Gene in verschiedenen Mikroorganismen exprimiert werden Als Vektoren eigenen sich sowohl Vektoren mit spezifischem Wirtsspektrum als auch Vektoren mit breitem Wirtsspektrum ("broad host range") Beispiele fur Vektoren mit spezifischem Wirtsspektrum, z. B. für E. coli sind pBR322 (Bolivar et al., Gene, 2, 95 - 113), der handelsübliche pBLUESCRIPT-KS+^{®}, pBLUESCRIPT-SK+^{®} (Stratagene), pUC18/19 (Yanisch-Perron et al., Gene 33, 103 - 119, 1985), pK18/19 (Pridmore, Gene, 56, 309 - 312, 1987), pRK290X (Alvarez-Morales et al., Nucleic Acids Research, 14, 4207 - 4227) und pRA95 (erhältlich von Nycomed Pharma AS, Huidove, Dänemark). Vorzugsweise wird pBLUESCRIPT-KS+^{®} angewendet.

Als "broad host range" Vektoren konnen alle Vektoren eingesetzt werden, die für Gramnegative Bakterien geeignet sind.
Beispiele für solche "broad host range" Vektoren sind pRK290 (Ditta et al., PNAS, 77, 7347 - 7351, 1980) oder deren Derivate, pKT240 (Bagdasarian et al., Gene, 26, 273 - 282, 1983) oder dessen Derivate, pGSS33 (Sharpe, Gene, 29, 93 - 102, 1984), pVK 100 (Knauf und Nester, Plasmid, 8, 45 - 54, 1982) bzw dessen Derivate, pME285 (Haas und Itoh, Gene, 36, 27 - 36, 1985) bzw dessen Derivate

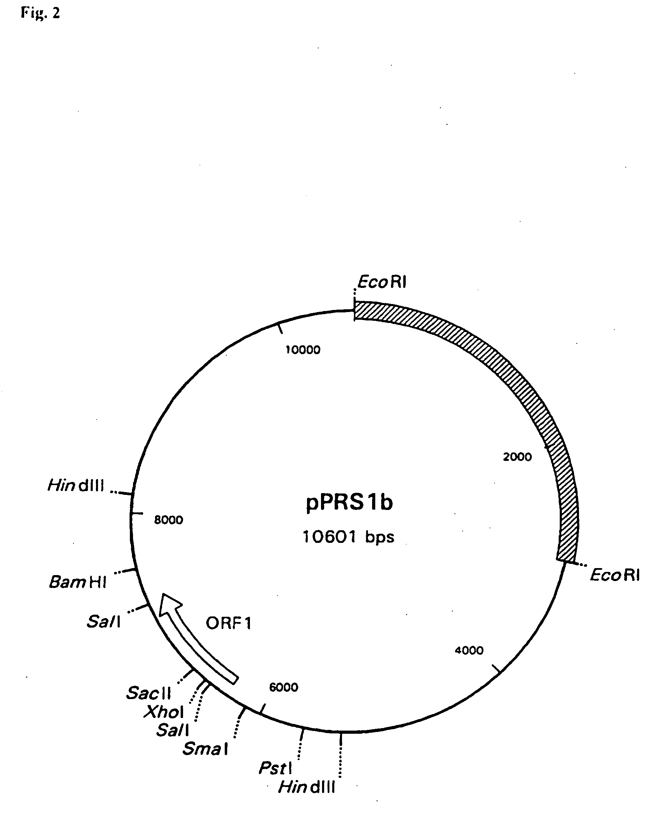

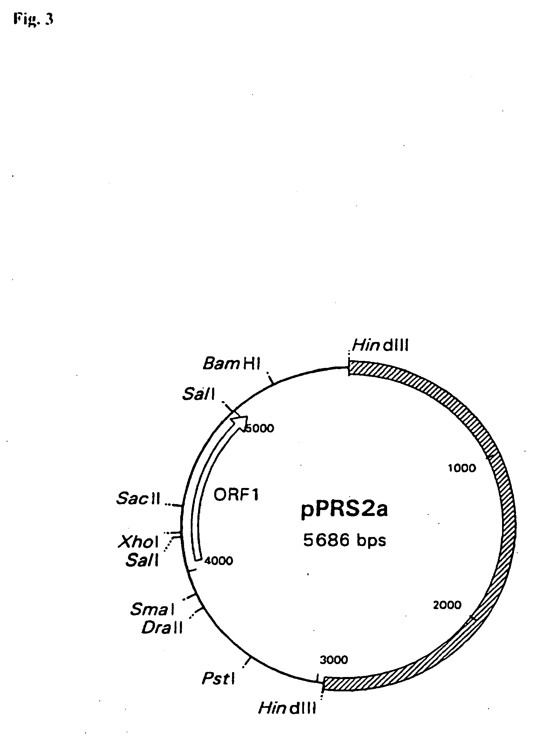

Auf diese Weise wurden beispielsweise die Plasmide pPRS1b (Fig. 2), pPRS2a (Fig. 3), pPRS4 und pPRS7 erhalten.

Zur Herstellung der Produktionsstamme fur die Fermentation, d. h Stamme, die für die Herstellung von z. B (R)-2,2-HTFMPS eingesetzt werden konnen, müssen die erfindungsgemassen DNA-Fragmente bzw. Vektoren in die gewunschten und zur Expression geeigneten Wirtsstamme eingebracht werden. Zweckmässig werden die Mikroorganismen hierzu in üblicher und an sich bekannter Weise mit den die erfindungsgemässen DNA-Fragmente enthaltenden Vektoren transformiert. Die Mikroorganismen können das erfindungsgemässe DNA-Fragment dann entweder auf einem Vektormolekül oder integriert in ihrem Chromosom enthalten.

Geeignete Wirtsstämme, vorzugsweise Stamme mit hoher Substrat- und Edukt-Toleranz sind beispielsweise Mikroorganismen der Gattung Pseudomonas, Comamonas, Bacillus, Rhodococcus, Acinetobacter. Rhizobium, Agrobacterium, Rhizobium / Agrobacterium oder Escherichia, wobei letztere bevorzugt sind Besonders bevorzugt sind die Mikroorganismen Escherichia coli DH5, Escherichia coli XL1-Blue^{®} und Escherichia coli XL1-Blue MRF'^{®} Geeignete Produktionsstamme sind somit beispielsweise Mikroorganismen der Spezies Escherichia coli DH5 und Escherichia coli XL1-Blue MRF'^{®}, jeweils enthaltend Plasmid pPRS1b, pPRS2a, pPRS4 oder pPRS7.

Der Mikroorganismus Escherichia coli XL1-Blue MRF'^{®}/pPRS2a wurde am 30 06. 1997 unter der Bezeichnung DSM 11635 bei der Deutschen Sammlung für Mikroorganismen und Zellkulturen GmbH, D-38124 Braunschweig, Mascheroderweg 1b, gemäss Budapester Vertrag hinterlegt.

Die Isolierung der transformierten Wirtsstamme (Produktionsstamme) kann aus einem selektiven Nährmedium erfolgen, dem ein Antibiotikum zugesetzt wird, gegen das die Stämme durch ein auf dem Vektor oder DNA-Fragment befindliches Markiergen resistent sind.

Das erfindungsgemasse Verfahren zur Herstellung von (S)- oder (R)-2,2-HTFMPS der Formeln 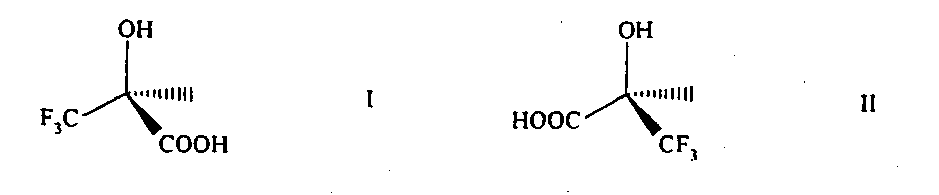 und / oder von (R)- oder (S)-2,2-HTFMPA der Formeln 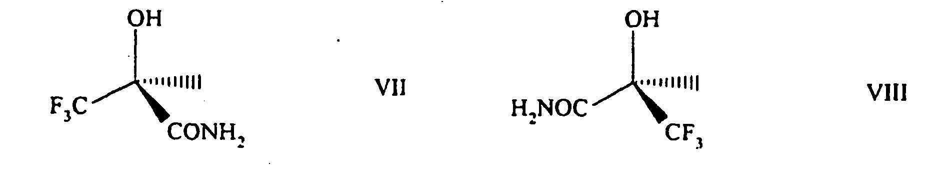 umfasst die Umsetzung des Propionsaureamids der Formel 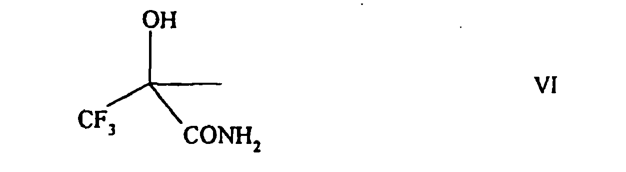 mittels den bereits beschriebenen erfindungsgemässen Mikroorganismen bzw. mittels aus den von ihnen isolierten Enzymen mit siereospezifischer Amidohydrolase-Aktivität.

Zweckmässig wird dabei das Verfahren zur Herstellung von (R)-2,2-HTFMPS und / oder von (S)-2,2-HTFMPA mittels den "Wildstämmen der Gattung Klebsiella, bevorzugt der Spezies Klebsiella oxytoca PRS 1 (DSM 11009), Klebsiella oxytoca PRS 1K17 (DSM 11623), Klebsiella planticula ID-624 (DSM 11354), Klebsiella pneumoniae ID-625 (DSM 11355), mittels den von diesen "Wildstämmen" abgeleiteten gentechnologisch veränderten Mikroorganismen oder mittels des Enzyms mit einer stereospezifischen Amidohydrolase durchgeführt.

Das Verfahren zur Herstellung von (S)-2,2-HTFMPS und / oder (R)-2,2-HTFMPA wird zweckmässig mittels den "Wildstämmen" der Spezies Pseudomonas sp. (DSM 11010), Rhodococcus opacus ID-622 (DSM 11344), Arthrobacter ramosus ID-620 (DSM 11350) und Bacillus sp. ID-621 (DSM 11351) durchgeführt.

Die Biotransformation kann nach üblichem Anzüchten der Mikroorganismen mit ruhenden Zellen (nicht wachsende Zellen, die keine Kohlenstoff- und Energiequelle mehr benötigen) oder mit wachsenden Zellen durchgeführt werden. Vorzugsweise wird die Biotransformation mit ruhenden Zellen durchgeführt.

Für die Biotransformation können fachmännisch übliche Medien eingesetzt werden, wie bspw. niedermolare Phosphatpuffer. HEPES-Puffer oder das zuvor beschriebene Mineralsalzmedium.

Zweckmässig wird die Biotransformation unter einmaliger oder kontinuierlicher Zugabe vom Propionsäureamid (Formel VI) so durchgeführt, dass die Konzentration 10 Gew.%, vorzugsweise 2,5 Gew.% nicht übersteigt.

Der pH-Wert des Mediums kann in einem Bereich von 4 bis 10, vorzugsweise von 5 bis 9,5 liegen. Zweckmässig wird die Biotransformation bei einer Temperatur von 10 bis 60 °C, vorzugsweise von 20 bis 40 °C, durchgeführt.

Die auf diese Weise erhaltene (S)- oder (R)-2,2-HTFMPS bzw. das (S)- oder (R)-2,2-HTFMPA kann durch übliche Aufarbeitungsmethoden wie z. B. durch Extraktion isoliert werden.

Durch Variation der Nährstoffe im Medium und durch Anpassen der Fermentationsbedingungen an den jeweiligen Mikroorganismus in üblicher Weise kann die Ausbeute an (S)-oder (R)-2,2-HTFMPS bzw an (S)- oder (R)-2,2-HTFMPA weiter verbessert werden.

Gegebenenfalls wird das (S)- oder (R)-2,2-HTFMPA entweder chemisch in Gegenwart einer Base oder mikrobiologisch mittels Mikroorganismen der Gattung Rhodococcus zur entsprechenden Saure hydrolysiert

Als Base kann ein Alkalimetallhydroxid eingesetzt werden. Als Alkalimetallhydroxid wird zweckmassig Natrium- oder Kaliumhydroxid eingesetzt.

Zweckmassig erfolgt die mikrobiologische Hydrolyse mit Mikroorganismen der Spezies Rhodococcus equi, Rhodococcus rhodochrous oder Rhodococcus sp. S-6 Vorzugsweise mit Mikroorganismen der Spezies Rhodococcus equi TG 328 (DSM 6710) bzw dessen funktionell aequivalente Varianten und Mutanten Der Mikroorganismus Rhodococcus equi TG 328 ist in der US-PS 5 258 305 beschrieben und wurde am 13 09 1991 bei der Deutschen Sammlung von Mikroorganismen und Zellkulturen GmbH, D-38124 Braunschweig, Mascheroderweg 1b, gemäss Budapester Vertrag hinterlegt.
Üblicherweise werden diese Mikroorganismen vor der eigentlichen mikrobiologischen Hydrolyse entsprechend Gilligan et al (Appl. Microbiol. Biotech., 39, 1993, 720 - 725) angezüchtet Die mikrobiologische Hydrolyse erfolgt im Prinzip nach fachmannisch üblichen Methoden Zweckmassig wird die Hydrolyse bei einer Temperatur von 20 bis 40 °C und bei einem pH von 6 bis 9 durchgefuhrt

Die Herstellung des Propionsaureamids der Formel 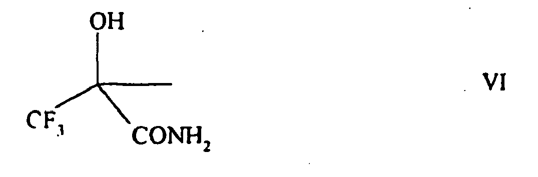 erfolgt derart, dass man zunachst in der ersten Stufe Trifluoracetessigester der Formel 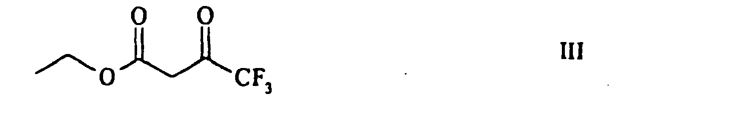 mit einer Mineralsaure in Trifluoraceton der Formel 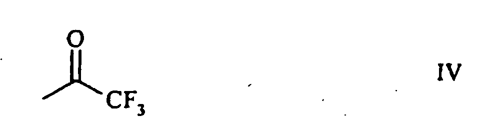 überführt.

Als Mineralsäure kann beispielsweise Salzsäure, Schwefelsäure, Salpetersäure oder Phosphorsäure verwendet werden. Vorzugsweise wird Schwefelsäure, Phosphorsäure oder Salpetersäure, insbesondere Schwefelsäure, verwendet.

Zweckmässig wird die Umsetzung in der ersten Stufe in einem polar protischen Lösungsmittel wie z. B. in einem niederen Alkohol, in Wasser oder in einer niederen Alkohol-Wasser-Mischung durchgefuhrt. Als niederer Alkohol kann beispielsweise Methanol, Ethanol, Propanol, Isopropanol, Butanol, tert-Butanol oder Isobutanol eingesetzt werden.

Die Umsetzung in der ersten Stufe wird zweckmassig bei einer Temperatur von 50 bis 100°C, vorzugsweise bei einer Temperatur von 70 bis 95 °C, durchgeführt.

In der zweiten Stufe des erfindungsgemässen Verfahrens wird Trifluoraceton (Formel IV) mit einem Cyanid in das Propionsäurenitril der Formel 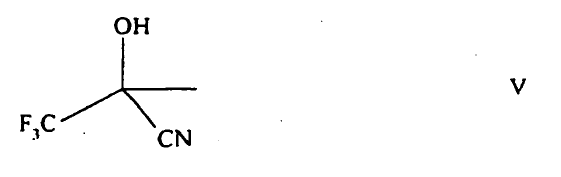 umgesetzt.

Zweckmässig wird als Cyanid ein Alkalimetallcyanid wie Natrium- oder Kaliumcyanid, vorzugsweise Natriumcyanid, eingesetzt.

Die Umsetzung in der zweiten Stufe wird zweckmässig in Gegenwart einer Mineralsäure durchgeführt. Als Mineralsäure können die gleichen wie die zuvor beschriebenen verwendet werden. Vorzugsweise wird als Mineralsäure Schwefelsäure eingesetzt
Üblicherweise wird die Mineralsäure im Überschuss bezogen auf Trifluoraceton eingesetzt. Vorzugsweise werden von 1 bis 10 mol Mineralsäure pro mol Trifluoraceton verwendet Als Lösungsmittel können die gleichen wie die in der ersten Stufe verwendet werden

Zweckmassig wird die zweite Stufe bei einer Temperatur von -20 bis 100 °C, vorzugsweise von 0 bis 20 °C durchgeführt.

In der dritten Stufe des erfindungsgemässen Verfahrens wird das Propionsäurenitril der Formel V entweder chemisch in einer konzentrierten Mineralsaure oder mikrobiologisch mittels mutierten Mikroorganismen der Gattung Rhodococcus in das Propionsäureamid der Formel VI überfuhrt.

Als Mineralsäuren konnen die gleichen wie die in der ersten und zweiten Stufe eingesetzt werden. Unter einer "konzentrierten Mineralsaure" wird im folgenden eine 30 bis 100%ige Mineralsaure verstanden Zweckmässig wird in der dritten Stufe eine 75 bis 100%ige, vorzugsweise eine 90 bis 100%ige Mineralsaure verwendet
Die chemische Umsetzung in der dritten Stufe wird zweckmassig bei einer Temperatur von 0 bis 160 °C, vorzugsweise von 70 bis 120 °C, durchgefuhrt

Die mutierten Mikroorganismen der Gattung Rhodococcus enthalten keine Amidase mehr und sind somit nicht mehr befahigt ein Amid in die entsprechende Säure zu überfuhren Die Mutation kann nach bekannten Methoden durchgefuhrt werden (J H. Miller, Experiments in Molecular Genetics, Cold Spring Harbor Laboratory, 1972, S 24) Zweckmässige Mutationsmethoden sind die Frameshift-Methode, Deletionsmethode oder Transposon-Insertionsmethode

Geeignete Mikroorganismen-Spezies für die Mutation sind Rhodococcus equi, Rhodococcus rhodochrous oder Rhodococcus sp. S-6 Vorzugsweise wird der zuvor beschriebene Rhodococcus equi TG 328 (DSM 6710) mutiert, wobei Rhodococcus equi TG 328-2 (DSM 1 1636) bzw dessen funktionell aequivalente Varianten und Mutanten erhalten wird. Der Mikroorganismus TG 328-2 wurde am 30 06 1997 bei der Deutschen Sammlung für Mikroorganismen und Zellkulturen GmbH, D-38124 Braunschweig, Mascheroderweg 1b, gemäss Budapester Vertrag hinterlegt Dieser Mikroorganismus wird unter den gleichen Bedingungen kultiviert wie die bereits zuvor beschriebenen nicht mutierten Mikroorganismen

Das (R)- und (S)-2,2-HTFMPA sind in der Literatur noch nicht beschriebene Verbindungen und daher ebenfalls Bestandteil der Erfindung. Diese können als neue Zwischenprodukte zur Herstellung von (R)- oder (S)-2,2-HTFMPS, z B. durch Hydrolyse in Gegenwart einer Base, eingesetzt werden

### Beispiel 1

### Herstellung von Trifluoraceton

500 g (4,9 Mol) konzentrierte Schwefelsäure (96%ig; Merck) wurden zu 11 destilliertem Wasser gegeben und das Ganze auf 73 °C erhitzt. Dann wurden 500 g (2,69 Mol) Trifluoracetessigester langsam hinzugefügt wobei sich zwei Phasen bildeten. Der Reaktionsansatz wurde bis zur Ruckflusstemperatur erhitzt und das dabei gebildete Trifluoraceton abdestilliert Nach 2 h wurden 293,8 g Trifluoraceton als farblose Flüssigkeit, entsprechend einer Ausbeute von ca. 90%, isoliert. Die GC-Analyse zeigte eine Reinheit von 92,1 %.

### Beispiel 2

### Herstellung von 2-Hydroxy-2-methyl-3,3,3-trifluormethylpropionsäurenitril

39,4 g Natriumcyanid (0,763 Mol) wurden zu 174 ml destilliertem Wasser hinzugegeben und das Ganze auf-1 °C gekuhlt Anschliessend wurden 100 g Trifluoraceton (0,822 Mol) tropfenweise hinzugefugt, wobei sich das Reaktionsgemisch auf 6 °C erwärmte. Nach Beendigung der Trifluoraceton-Zugabe wurden bei 4 - 5 °C 293,4 g 6 N Schwefelsäure (1,4916 mol H') hinzugegeben Danach wurde das Reaktionsgemisch uber Nacht bei Raumtemperatur geruhrt. Anschliessend wurde mit Ethylether oder mit tert. Butylmethylether extrahiert und die vereinigten organischen Phasen wurden entweder unter Normaldruck bei 32 °C oder unter leichtem Vakuum (300 - 120 mbar) destilliert. Insgesamt wurden 88 g Produkt mit einer Reinheit von 91,2 % (gemessen mittels GC), entsprechend einer Ausbeute von 75.6 %, erhalten

### Beispiel 3

### a) Chemische Herstellung von (R,S)-2,2-HTFMPA

Unter Argon-Atmosphäre wurde 98 %ige Schwefelsäure vorgelegt Dazu wurden 15 g 2-Hydroxy-2-methyl-3,3,3-trifluormethylpropionsäurenitril (86,9 % gemäss GC) hinzugefugt und die Reaktion wurde auf 95 °C erhitzt. Nach Edukt-Zugabe wurde das Reaktionsgemisch fur 15 min auf 114°C erhitzt. Danach wurde das Reaktionsgemisch auf 5 °C abgekühlt, wobei sich eine dicke braune Lösung bildete. Anschliessend wurden 40 g destilliertes Wasser tropfenweise hinzugegeben. Dabei sollte sich das Reaktionsgemisch nicht über 15 °C erwarmen Die dabei gebildete gelbliche Suspension wurde 15 min lang auf -15 °C abgekuhlt und dann filtriert Der Filterkuchen wurde mit 20 ml eiskaltem Wasser gewaschen und dann im Vakuum getrocknet. Dabei wurden 12,64 g leicht gelbliches Roh-Produkt erhalten Anschliessend wurde das Roh-Produkt in 13 ml Ethylacetat zum Rückfluss erhitzt und dann auf Raumtemperatur abgekühlt Zu dieser Suspension wurden 15 ml Hexan hinzugegeben und das Ganze auf 0 °C abgekühlt Danach wurde nochmals mit Hexan gewaschen. Nach Trocknen im Vakuum wurden 11,8 g Produkt, entsprechend einer Ausbeute von 80,2 %, erhalten
Schmp 143,1 - 144,3°C

### b) Mikrobiologische Herstellung von (R,S)-2,2-HTFMPA (mittels mutiertem Mikroorganismus der Gattung Rhodococcus)

Zur Mutation wurde Rhodococcus equi TG 328 standardgemäss in "Nutrient Broth" mit Acridin ICR 191 uber Nacht bei 30 °C inkubiert Danach wurden die Zellen geerntet und mit 0.9%iger NaCl-Losung gewaschen. Dann wurden die Zellen in frischem Medium uber Nacht bei 30 °C inkubiert
Die Selektion der mutierten Zellen wurde in einem Mineralsalzmedum gemäss Gilligan et al (Appl Microbiol Biotech, 39, 1993, 720-725) in Gegenwart von Fluoracetamid als "counterselektives Agens" durchgefuhrt. Dieses "counterselektive" Agens tötet nur wachsende Bakterien ab. Die Mutanten, die keine Amidase mehr enthalten und nicht mehr mit (R,S)-2,2-HTFMPA wachsen, überleben und werden angereichert.
Anschliessend wurden die Zellen geerntet, mit 0,9%iger NaCl-Lösung gewaschen, in frischem Medium über Nacht inkubiert und dann ausplattiert Die Kolonnien wurden auf Nitrilhydratase-Aktivitat getestet Die Häufigkeit der gewünschten Mutation war 2%.

Die Mutante von Rhodococcus equi TG 328-2 wurde in einem Mineralsalzmedium gemäss Gilligan et al, (ibid) angezuchtet. Die gewaschenen Zellen wurden bei einer OD₆₅₀ₙₘ = 5,0 sowohl mit 2-Hydroxy-2-methyl-3,3,3-trifluormethylpropionsaurenitril-Lösung (1%) als auch mit einer (R,S)-2,2-HTFMPA-Lösung (1%) in 100 mM Phosphatpuffer (pH 7,7) bei 37 °C inkubiert. Nach 16 h wurde mittels GC-Analyse gezeigt, dass das Nitril quantitativ zum Amid umgesetzt wurde, wogegen das Amid nicht zur Säure hydrolysiert wurde.

### Beispiel 4

### Herstellung von (S)-2,2-HTFMPA und (R)-2,2-HTFMPS mittels eines Mikroorganismus enthaltend eine Amidohydrolase (Wildstamm)

### 4.1. Selektion und Isolation von Mikroorganismen mit (R)- und (S)-Amidase-Aktivität

Zu 10 g Bodenprobe wurde 100 ml Phosphatpuffer (0, 1 M, pH 7,0) hinzugeben und das Ganze 10 Minuten stehen gelassen und filtriert. Danach wurde der Überstand (5,0 ml) oder 1 ml Abwasser (ARA, Visp) in ein Mineralsalzmedium (25 ml; Kulla et al., Arch. Microbiol. 135, S 1 - 7, 1983) überimpft, welches Glycerin und (R,S)-HTFMPA (Kohlenstoff-/Stickstoff-Verhältnis 5·1) enthielt. Anschliessend wurde diese Kultur inkubiert, bis eine Mischkultur entstanden war, die (R)- und / oder (S)-2,2-HTFMPA als einzige Stickstoff-Quelle benutzen kann Diese Kultur wurde dann mehrmals überimpft und bei 30 °C bebrütet bis eine Mischkultur entstanden war.
Die Reinkultur dieser Mikroorganismen wurde unter Zuhilfenahme traditioneller mikrobiologischer Techniken erhalten
Die auf diese Weise erhaltenen Mikroorganismenstämme wurden dann auf Agar-Platten fur ihr Wachstum auf (R,S)-2,2-HTFMPA getestet Die positiven Stämme wurden weiter getestet. Mit diesen Stämmen wurde dann ein Vorkultur-Medium angeimpft. Die in dieser Vorkultur enthaltenen Mikroorganismen wurden ins Mineralsalzmedium überführt und dann auf ihre Fähigkeit überpruft, selektiv (R)-2,2-HTFMPA und / oder (S)-2,2-HTFMPA als einzige Stickstoff-Quelle zu nutzen, wobei der Uberstand mittels GC auf die Bildung von (R)-2,2-HTFMPS oder (S)-2,2-HTFMPS und auf die Anreicherung eines der beiden Amid-Enantiomere gepruft wurde

### 4.2. Aktivitätsbestimmung der (R)- oder (S)-2,2-HTFMPA-Amidohydrolase

Zur Aktivitätsbestimmung der Hydrolasen wurde die Mikroorganismensuspension auf eine optische Dichte von 4,0 bei 650 nm eingestellt. Als Medium diente ein Phosphatpuffer (100 mmolar), pH 7,0, mit 0,5 Gew % (R,S)-HTFMPA Diese Suspension wurde 2 h bei 30 °C unter Schütteln inkubiert Das durch die Hydrolase freigesetzte NH₄' wurde entweder kolorimetrisch oder mittels einer Ammonium-Elektrode bestimmt und das HTFMPA wurde mittels GC gemessen. Die Aktivität wurde als g (R)- oder (S)-HTFMPA umgesetzt/l/h/optische Dichte bei 650 nm ausgedruckt, vorausgesetzt, dass 1 mmol gebildetes NH4' = 1 mmol umgesetztem HTFMPA entspricht.

**Tabelle 1: Die Hydrolase-Aktivität von Klebsiella und Pseudomonas**

| Stamm | Hydrolase-Aktivitat | |
|---|---|---|
| | (R)-spezifische | (S)-spezifische |
| | (g/l/h/O D. 650 nm) | |
| DSM 11009 | 0,11 | - |
| (Klebsiella oxytoca PRS1) | | |
| | | |
| DSM 11010 | - | 0,09 |
| (Pseudomonas sp) | | |

### 4.3. Herstellung von (S)-2,2-HTFMPA und (R)-2,2-HTFMPS.

Klebsiella oxytoca PRS 1 (DSM 11009), Klebsiella planticula ID-624 (DSM 11354) oder Klebsiella pneumoniae ID-625 (DSM 11355) wurden auf Mineralsalzmedium-Agar-Platte mit Glycerin als Kohlenstoff-Quelle und (R,S)-2,2-HTFMPA als einzige Stickstoff-Quelle 2 Tage lang bei 30 °C bebrutet Die Zusammensetzung des Mineralsalzmediums ist in Kulla et al, Arch Microbiol , 135, S 1 - 7, 1983 beschrieben Mit diesen ausplattierten Mikroorganismen wurde ein Vorkultur-Medium mit der gleichen Zusammensetzung beimpft und 2 Tag lang bei 30 °C inkubiert
Das gleiche Mineralsalzmedium (600 ml) wurde mit 50 ml Vorkultur zur Induktion und Biomassen-Produktion beimpft und bei 30 °C 21 h lang inkubiert Anschliessend wurden die Zellen durch Zentrifugation geerntet und in 0,1 M Phosphatpuffer , pH 7.0 aufgenommen Nach Resuspension der Zellen in 0,05M Phosphat Puffer (500 ml, pH 8.0) wurde ein optische Dichte bei 650 nm von 10 eingestellt und 1,0 Gew % (R,S)-2,2-HTFMPA zugefugt. Nach einer Inkubation von ca 5,5 h bei 40 °C wurde (R)-2,2-HTFMPA vollständig zur entsprechenden Saure umgesetzt, was einer optischer Reinheit (ee) von 100% und einer Ausbeute von 48%.
Der Reaktionsablauf wurde anhand der NH₄'-Freisetzung und anhand von GC-Analyse des Überstandes

### 4.4. Herstellung von (S)-2,2-HTFMPS und (R)-2,2-HTFMPA mittels eines Mikroorganismus enthaltend eine (S)-Amidohydrolase

In analoger Weise zu Beispiel 4.1. wurde der Mikroorganismen Pseudomonas sp. (DSM 11010), Rhodococcus opacus ID-622 (DSM 11344), Arthrobacter ramosus ID-620 (DSM 11350) und Bacillus sp ID-621 (DSM 11351) isoliert. Die Induktionsdauer betrug 2 Tage unter ansonst gleichen Bedingungen wie Beispiel 4.3..
Im Gegensatz zu Beispiel 4.3 wurde die Biotransformation bei diesen Mikroorganismen mit 0,5 Gew.% (R,S)-2,2-HTFMPA durchgeführt. Der Stamm Pseudomonas sp. (DSM 11010) besitzt eine (S)-spezifische Hydrolase und die Aktivität der Hydrolase wurde bei pH 6,0 zu 0,09 g (S)-2,2-HTFMPA (ee = 86%), umgesetzt/l/h/O.D 650 nm bestimmt.

### 4.5. Aufarbeitung von (S)-2,2-HTFMPA und (R)-2,2-HTFMPS

### a) mittels Extraktion

196 ml einer Reaktionsmischung enthaltend (S)-2,2-HTFMPA und (R)-2,2-HTFMPS (erhalten aus Beispiel 4.3) 0,1 M Phosphatpuffer (250 ml), pH 10, wurden 3 mal mit Ethylacetat (200 ml) extrahiert. Die vereinigten organischen Phasen wurden mit Na₂SO₄ getrocknet und dann bei 40 °C und 50 mbar eingedampft. Auf diese Weise wurden 912 mg feuchtes Produkt erhalten. Dieses wurde in heissem Ethylacetat (1,3 ml) gelöst und die Losung dann auf Raumtemperatur abgekühlt. Nach Zugabe von Hexan (2 ml) fiel das Produkt aus Die Mischung wurde auf 0,°C abgekühlt, das Produkt filtriert und dann im Vakuum bei 50 °C getrocknet Dabei wurden 791 mg des (S)-2,2-HTFMPA erhalten, entsprechend einer Ausbeute von 78,2 % bez. der halben eingesetzten Menge. Mittels chiraler GC-Analyse wurde nur das (S)-Isomere identifiziert
Die verbleibende Wasserphase wurde mit konz. HCl auf pH 1 eingestellt und dann 2 mal mit Ethylacetat (200 ml) extrahiert. Die Extrakte wurden bei 40 °C eingedampft und dann getrocknet Dann wurde 1 ml Toluol hinzugegeben und das Ganze auf Raumtemperatur abgekuhlt. Es wurden nochmals 2 ml Hexan hinzugegeben und das Ganze auf 0 °C gekuhlt. Der Feststoff wurde 2 - 3 mal mit Hexan gewaschen und dann getrocknet Insgesamt wurden aus der Wasserphase nach Trocknen im Vakuum bei 3 5 °C 664 mg (R)-2,2-HTFMPS erhalten, entsprechend einer Ausbeute von 65,7 % bez der halben eingesetzten Menge Mittels chiraler GC-Analyse wurde nur das (R)-Isomere identifiziert

### b) mittels Elelctrodialyse (direkte Isolation von (S)-HTFMPS

Eine Reaktionsmischung enthaltend (S)-2,2-HTFMPA und (R)-2,2-HTFMPS (erhalten aus Beispiel 4.3) wurde der Ultrafiltration unterworfen, um zellulares Material zu entfernen. Die daraus resultierende Losung wurde der Elektrodialyse unterworfen Dabei wanderte das (R)-2,2-HTFMPS und alle Puffersalze durch die Membran hindurch. Nach Beendigung der Elektrodialyse wurde eine Lösung von reinem (S)-2,2-HTFMPA (2342,2 g) erhalten. Diese Lösung wurde bei 135 °C und 20 mbar destilliert bis 447 g Produkt erhalten wurden Dann wurden 32,7 g festes NaOH 0,8 mol) hinzugegeben und die Reaktionsmischung wurde für 3 h auf Rückflusstemperatur erhitzt Nach dieser Zeit war das (S)-2,2-HTFMPA vollständig zur (S)-2,2-HTFMPS umgesetzt. Die Losung wurde auf eine Temperatur von unterhalb 25 °C abgekühlt und der pH mit 93,6 g konz HCl von pH 13,8 auf pH 1,0 eingestellt. Die wassrige Phase wurde 2mal mit Acetessigester (500 ml) extrahiert Die vereinigten organischen Phasen wurden mit Na₂SO₄ getrocknet und dann filtriert. Die Losung wurde am Rotationsverdampfer bis zu einer dicken Suspension eingedampft. Zur Suspension gab man 2mal je 20 ml Toluol. um dann die dabei erhaltene Suspension nochmals einzuengen Dann wurden nochmals 10 ml Toluol hinzugefügt, um das Ganze zum Rückfluss zu erhitzen Die Losung wurde auf Raumtemperatur abgekühlt und Hexan (30 ml) wurde hinzugegeben bis das Produkt prazipitierte. Die Suspension wurde auf -10°C abgekühlt und das Produkt mittels Ultrafiltration gesammelt Nach Trocknen im Vakuum (Temperatur < 35 °C) wurden 14,1 g (0,0892 mol) reine (S)-2,2-HTFMPS (ee-Wert 99,7%) entsprechend einer Ausbeute von 35% (berechnet ausgehend von der Hälfte des Eduktes). erhalten

### Beispiel 5

### a) Chemische Hydrolyse von (S)-2,2-HTFMPA zu (S)-2,2-HTFMPS

0,47 g Natriumhydroxid (11,6 mMol) wurden in 5 ml destilliertes Wasser gegeben Hierzu wurden 650 mg (4,14 mMol) (S)-2,2-HTFMPA hinzugefügt und das Ganze auf Rückflusstemperatur erhitzt Nach 2 h wurde die Reaktionsmischung auf Raumtemperatur abgekuhlt und der pH mit 10%iger HCl auf pH 1,0 eingestellt. Anschliessend wurde die Mischung 2 mal mit Ethylacetat (10 ml) extrahiert. Die vereinigten organischen Phasen wurden uber Na₂SO₄ getrocknet, filtriert und bei maximal 40 °C eingedampft Nach Trocknen im Vakuum-Ofen (45 min bei 35 °C) wurden 618 mg (S)-2,2-HTFMPS, entsprechend einer Ausbeute von 94,4 %, erhalten. Mittels chiraler GC-Analyse wurde nur das eine Isomere identifiziert

### b) Mikrobiologische Hydrolyse von (S)-2,2-HTFMPA zu (S)-2,2-HTFMPS

Rhodococcus equi TG 328 (DSM 6710) wurden in einem Mineralsalzmedium gemäss Gilligan et al., (ibid) ängezüchtet. Die gewaschenen Zellen mit einer OD₆₅₀ₙₘ = 5,0 wurden mit einer (S)-2,2-HTFMPA-Lösung (1% in 100 mM Phosphatpuffer, pH 7,7) bei 37°C inkubiert. Nach 16 h wurde mittels GC-Analyse gezeigt, dass das (S)-2,2-HTFMPA quantitativ in die (S)-2,2-HTFMPS überfuhrt wurde.

### Beispiel 6

### 6.1 Erzeugung eines Kapsel-negativen Mutanten von Klebsiella oxytoca PRS1

Klebsiella oxytoca PRS 1 bildete eine Schleimkapsel, die dem Stamm ungünstige Eigenschaften bei der Fermentation verlieh. Ein Kapsel-negativer Stamm war vorteilhaft für die Zellabtrennung und die nachfolgende Aufarbeitung.
Kapsel-negative Mutanten wurden mittels Acridine ICR 191 (J. H Miller Experiments in Molecular Genetics, Cold Springs Harbor, 1972), wie nachfolgend beschrieben, isoliert.

Klebsiella oxytoca PRS 1 wurde im Mineralsalzmedium enthaltend 0,2% Glucose und in Anwesenheit von Acridine ICR 191 angeimpft und uber Nacht bei 30 °C bebrütet. Anschliessend wurde diese Kultur in frisches Medium überimpft und nochmals über Nacht bei 30 °C bebrutet Die Kultur wurde verdunnt und auf Nutrient Agar ausplattiert Nicht schleimige Kolonien wurden gepickt und uberpruft Die Mutanten wurden mit einer Frequenz von 0,18% isoliert. Ein Beispiel solcher Mutanten ist Klebsiella oxytoca PRSIK17 (DSM 11623) Diese Mutante zeigte das gleiche Wachstumsverhalten wie der Wildtyp Das (R)-spezifische Enzym besitzt die gleiche Aktivitat wie in Klebsiella oxytoca PRS 1 aber der Stamm bildete keine Schleim-Kapsel. Diese Mutante wurde für die Enzymcharakterisierung und die Genklonierung benutzt

### 6.2 Präparation chromosomaler DNA von Klebsiella oxytoca PRS1K17 (Kapsel-negative Mutante von PRS1)

Die chromosomale DNA einer frischen Übernachtkultur von Klebsiella oxytoca PRS1K17 (100 ml Nutrient Yeast Broth, 30 °C) wurde nach der modifizierten Methode von R H Chesney et al (J. Mol. Biol, 130, 1979), 161 - 173) isoliert:
Die abzentrifugierten Zellen (15 min, 6'500 x g, 4 °C) wurden in Tris-Puffer (2,25 ml, 0,05 mol/l, pH 8,0, 10% (w/v) Saccharose resuspendiert

Nach Zugabe von 375 µl Lysozymlosung (10 mg/ml; 0,25 mol/l Tris-HCl-Puffer, pH 8.0) und 900 µl 0, 1 mol/l EDTA, pH 8,0, wurde die Suspension fur 10 min auf Eis gekühlt Darauf folgte die Zugabe von 450 µl 5% (w/v) SDS und von 50 µl Ribonuklease (10 mg/ml H₂O) und eine Inkubation bei 37 °C für 30 min. Die Inkubation wurde nach Zugabe einer Spatelspitze Proteinase K und 400 µl Pronase (20 ml/ml H₂O) für 2 h fortgesetzt.
Es wurde nach Mischen mit 4,3 g CsCl zentrifugiert (30 min, 40'000 x g, 20 °C), mit 250 µl Ethidiumbromid (10 mg/ml) versetzt und in der Ultrazentrifuge (Vti 65.2-Röhrchen) zentrifugiert (mehr als 8 h, 246'000 x g, 20 °C) Unter langwelligem UV-Licht wurde die DNA-Bande aus dem Röhrchen abgesaugt. Nach Zugabe des 4-fachen Volumens TE-Puffer (10 mmol/l Tris-HCl, pH 8,0, 1 mmol/l EDTA) wurde das Ethidiumbromid dreimal mit Wasser gesattigtem n-Butanol extrahiert Die DNA wurde mit Isopropanol prazipitiert, in TE-Puffer aufgenommen und 15 min bei 65 °C inkubiert Das Praparat konnte bei 4 °C aufbewahrt werden

### 6.3 Restriktion und Ligation der chromosomalen DNA

5 µg Klebsiella oxytoca PRS1K17-DNA und 4,5 µg Vektor-DNA (pBLUESCRIPT-KS+^{®}) wurden jeweils mit 20 Units Restriktionsenzym HindIII in einem Totalvolumen Restriktionspuffer von 100 µl geschnitten (6,5 h bei 37 °C) Die DNAs wurden mit Ethanol präzipitiert und im Speed Vac^{R} Concentrator getrocknet Die Niederschlage wurden im Ligationspuffer (20 mmol/l Tris-Puffer, 10 mmol/l DTT (Dithiothreitol), 10 mmol/l MgCl₂, 0,6 mol/l ATP (Adenosintriphosphat, pH 7,2) aufgenommen und vereinigt (Ligationsvolumen 100 µl)
Nach Zugabe von 1 Unit T4-DNA-Ligase wurde über Nacht bei 13 °C inkubiert
Die DNA des Ligationsgemisches wurde mit Isopropanol prazipitiert und in 30 µl Wasser zur Transformation aufgenommen.

### 6.4 Transformation von E. coli XL1-Blue MRF'^{®} und Selektion

Kompetente Zellen von E coli XL1-Blue MRF'^{®} wurden mittels Elektroporation mit dem Ligationsgemisch nach der beschriebenen Methode von S. Fiedler und R Wirth (Analyt Biochem., 170, 1988, 38-44) transformiert
Zum Plasmidnachweis wurde auf Nutrient Agar mit Ampicillin (100 µg/ml) und zum "Insert"-Nachweis mit 0,5 mmol/l IPTG (Isopropyl-β-D-thiogalaktiosid) und X-Gal (30 µ g/ml, 5-Brom-4-chlor-3-indolyl-β-D-galaktopyranosid) bei Inkubation bei 37 °C selektioniert
Bei einer Transformationsfrequenz von 1,7 x 10⁸ cfu/ml ("colony forming units" ≙ lebenden Zellen) besassen nahezu alle Klone ein HindIII "Insert"

### Beispiel 7

### Screening der Klebsiella oxytoca PRS1K17-Genbank nach dem (R)-spezifischen Amidohydrolase-Gen

Klone mit Hybridplasmiden (HindIII "Insert") wurden auf Minimalmedium-Agar nach H. Kulla et al. (Arch. Mikrobiol., 135, 1983, 1-7) mit 0,4% (v/v) Glycerin als C-Quelle, 0,2% (w/v) (R,S)-2,2-HTFMPA als einzige N-Quelle und Ampicillin (5 µg/ml) zur Plasmidstabilisierung, auf ihre Wachstumsfähigkeit überpruft. Nur Klone, welche das intakte Amidohydrolase-Gen sad auf dem DNA-"Insert" im Plasmid enthielten, waren fähig, das (R,S)-HTFMPA als N-Quelle zu verwerten, dieses in die gesuchte (R)-Säure umzusetzen und auf diesem Minimalmedium zu wachsen. Solchermassen selektionierte Klone enthielten alle ein Hybridplasmid aus Vektor pBLUESCRIPT-KS+^{®} mit einem HindIII "Insert" von ca. 2,73 kb

Auf diese Weise wurde der Stamm E coli XL1-Blue MRF'^{®} mit dem als pPRS2a bezeichneten Plasmid identifiziert, aus dem das Plasmid pPRS2a isoliert und näher charakterisiert wurde

### Beispiel 8

### Lokalisation des Amidohydrolase-Gens (sad) auf dem klonierten HindIII-Fragment

### 8.1 Restriktionskarte von pPRS2a

Durch Restriktionsanalyse nach herkommlichem Vorgehen (Current Protocols Molecular Biology, John Wiley and Sons, New York, 1987, Abschnitt 2, wurde eine grobe Restriktionskarte vom pPRS2a bezuglich XhoI, DraII, SmaI, Pstl, SalI, BamH1 erstellt. Fig. 1 zeigt die Restriktionskarte.

### 8.2 Formulierung von gemischten DNA-Oligomeren beruhend auf der N-terminalen Peptidsequenz der Amidohydrolase

Aufgrund des genetischen Codes konnte ein gemischtes DNA-Oligomer für die N-terminale Peptidsequenz der Klebsiella oxytoca PRS1K17 Amidohydrolase formuliert und mit einer DNA-Synthese-Maschine synthetisiert werden. AS = Aminosauresequenz

### 8.3 "Southern Blot-Hybridisierung" von Restriktionsfragmenten des Plasmids pPRS2a

Die über Agarosegel-Elektrophorese (0,6%) aufgetrennten DNA-Fragmente, die nach unterschiedlichen Restnktionen (BamHI, SmaI, DraII, HindIII, EcoRI) von pPRS2a erhalten wurden, wurden uber das bekannte "Southern Blot-Verfahren" auf Nitrocellulose übertragen (Current Protocols in Molecular Biology, John Wiley and Sons, New York, 1987, Abschnitt 29ff)
Die DNA-Oligomere wurden gleichermassen mit Digoxigenin 3'- endmarkiert
Die Hybridisierung gegen die "Southern Blots" erfolgte nach dem bekannten Vorgehen (in der oben genannten Literaturstelle)
Durch Hybridisierung gegen das der N-terminalen Proteinsequenz entsprechende Nukleotid-Oligomer konnte ein 1,44 kb grosses SmaI-BamHI-DNA-Fragment oder ein 1,52 kb grosses DraII-BamHI-DNA-Fragment auf dem Hybridplasmid pPRS2a markiert werden

### 8.4 Subklonierungen des Hydrolase-Gens (sad)

Das 1,52 kb grosse DraII-BamHI-DNA-Fragment, oder das 1,91 kb grosse PstI-BamHI-DNA-Fragment, welches fur die (R)-spezifische Amidohydrolase aus Klebsiella oxytoca PRS1K17 codiert, wurde in gleichermassen verdaute Vektor-DNA pBLUESCRIPT-KS+^{®} inseriert.
Der Vektor pBLUESCRIPT-KS+^{®} mit dem 1,52 kb grossen Drall-BamHI-DNA-Fragment wurde als Hybridplasmid pPRS7 bezeichnet Der Vektor pBLUESCRIPT-KS+^{®} mit dem 1,91 kb grossen PstI-BamHI-DNA-Fragment wurde als Hybridplasmid pPRS4 bezeichnet.

### 8.5 Sequenzierung des Hydrolase-Gens (sad)

Das weiter oben unter 8.3 beschriebene 1,44 kb große SmaI-BamHI-Fragment wurde mit Hilfe eines Laser-Fluoreszenz-DNA-Sequenators einer Fluoreszenz-Sequenziereung gemäß der modifizierten Didesoxymethode nach Sanger unterworfen. Auf diese Weise wurde die als SEQ ID No. 1 bezeichnete Nukleotidseqttenz bestimmt, aus der sich für die Amidohydrolase die separat unter SEQ ID No 2 dargestellte Aminosäuresequenz ableitet

### Beispiel 9

### Aktivitätsbestimmung der (R)-Amidohydrolase-Klone

Die Aktivitatsbestimmung wurde analog zu Beispiel 4 2 durchgefuhrt.
Die Ergebnisse mit E coli / pPRS 1b und E. coli / pPRS2a als Beispiel sind in Tabelle 2 zusammengestellt.

| Klon | Hydrolase-Aktivitat | | Stunden (h) |
|---|---|---|---|
| | (R)-Amid g/l | (S)-Amid g/l | |
| E. coli XL1-Blue MRF'^{®} / pPRS 1b (EcoRI-Klon) | 5,35 | 5,92 | 0 |
| E. coli XL1-Blue MRF'^{®} / pPRS1b (EcoRI-Kion) | 0,00 | 5.84 | 4 |
| | ∼ Anfangsaktivität (37°C) von 0,29g/l/h / OD₆₅₀ₙₘ | | |
| E coli XL1-Blue MRF'^{®} / pPRS2a (HindIII-Klon) | 5,66 | 5.92 | 0 |
| E. coli XL1-Blue MRF'^{®} / pPRS2a (HindIII-Klon) | 0,00 | 6.20 | 8 |
| | ∼ Anfangsaktivitat (37 °C) von 0,13 g/l/h / OD₆₅₀ₙₘ | | |

### Beispiel 10

### Enzymreinigung und Enzymcharakterisierung

### 10.1 Enzymreinigung

Während der Reinigung wurden die aktiven Fraktionen kolorimetrisch bestimmt. Danach wurde die Aktivität des zellfreien Extraktes und des reinen Enzyms mittels GC-Methode ermittelt. Zellen von Klebsiella oxytoca PRSI (200 ml; OD₆₅₀=21 in 100 mM Phosphatpuffer, pH 7,5) wurden durch 3maliges Passieren durch die French-Presse bei 19000 psi (1309 bar) aufgebrochen Benzonase (1 µl x 30 ml Extrakt⁻¹) wurde hinzugefügt und dann wurde das Extrakt für 15 min bei 100000 x g 1 h zentrifugiert. Der Uberstand (2,94 mg x ml⁻¹) wurde 10 min auf 80 °C erhitzt und dann das prazipitierte Protein durch Zentrifugation entfernt. Der Uberstand (170 ml. 0,83 mg x ml⁻¹) wurde auf eine HiLoad Q-Sepharose 26/10-Chromatographie-Saule (Pharmacia) aufgetragen, welche zuvor mit 50 mM Phosphatpuffer (pH 7,5, Puffer A) äquilibriert worden war. Ungebundenes Protein wurde mit 130 ml Puffer A von der Saule gewaschen. Dann wurde ein linearerer Gradient (500 ml. I M NaCl - 0 M NaCl in Puffer A) angelegt, wobei die Durchflussrate 2,5 ml x min⁻¹ betrug 5 ml Fraktionen wurden gesammelt und auf Aktivitat getestet. Die aktivsten Fraktionen (30 - 37; 40 ml) wurden vereinigt, mittels Ultrafiltration zu 7,5 ml konzentriert und dann wurde der Puffer gegen einen 10 mM Phosphatpuffer (pH 7,5) durch Gelfiltationschromatographie (Sephadex G-25 M, PD 10, Pharmacia) ausgetauscht. Anschliessend wurden die aktiven Fraktionen auf eine Hydroxyapatit-Saule (5 ml; Bio-Scale CHTI, BioRad), welche mit einem 10 mM Phosphatpuffer aquilibriert worden war, aufgetragen Mittels eines Gradienten (90 ml; 0,5 mM Phosphatpuffer - 10 mM Phosphatpuffer, pH 7.5) wurden 1 ml-Fraktionen bei einer Durchflussrate von 2,0 ml x min⁻¹ gesammelt und auf Aktivität getestet Die Fraktionen 17 - 25 und 32 - 34 hatten Aktivität. Das Protein (M, 37000) von Fraktion 19 und den Fraktionen 33 und 34 war gemäss SDS-PAGE rein. Das Protein von Fraktion 20 war mehr als 95 % rein Die Fraktionen 20 - 25 wurden vereinigt, auf 200 µl konzentriert und dann auf eine Gelfiltrationschromatographie-Säule (Superose 12, Pharmacia) aufgetragen Gemass SDS-PAGE waren die Fraktionen 23 - 26 rein

### 10.2 Proteinsequenzierung

Eine N-terminale Aminosauresequenz wurde vom Western-Blott erhalten, dann wurde das Protein mit Trypsin verdaut, die Peptide mittels HPLC isoliert und sequenziert.

| | |
|---|---|
| N-Terminus. | Met Lys Trp Leu Glu Glu Ser Ile Met Ala Lys Arg Gly Val Gly Ala Ser Arg Lys Pro (SEQ ID Nr 3) |
| T3 | Val Tyr Trp Ser Lys (SEQ ID Nr. 4) |
| T4: | Lys Pro Val Thr His His Leu Thr Glu Glu Met Gln Lys (SEQ ID Nr. 5) |
| T5. | Tyr Thr Val Gly Ala Met Leu Asn Lys (SEQ ID Nr. 6) |
| T6A: | Met Glu Asn Ala Glu Asn Ile Met Ser Ile Gly Ser Ala Arg (SEQ ID Nr. 7) |
| T7 | Trp Leu Glu Glu Ser Ile Met Ala Lys (SEQ ID Nr 8) |
| T8 | Met Pro Phe Leu Asn Pro Gln Asn Gly Pro Ile Met Val Asn Gly Ala Glu Lys (SEQ ID Nr 9) |
| T9-2: | Asp Ala Phe Glu Gly Ala Ile Asn Ser Glu Gln Asp Ile Pro Ser Gln Leu Leu Lys (SEQ ID Nr. 10) |
| T9-2: | Glu Phe His Tyr Thr Ile Gly Pro Tyr Ser Thr Pro Val Leu Thr Ile Glu Pro Gly Asp Arg (SEQ ID Nr 11) |
| T11. | Leu Phe Ile Gly Asp Ala His Ala Glu Gln Gly Asp Gly Glu Ile Glu Gly Thr Ala Val Glu Phe Ala (SEQ ID Nr. 12) |
| T13-1 | Gly Asp Val Leu Ala Val Tyr Ile Glu Ser Met Leu Pro Arg (SEQ ID Nr 13) |
| T13-2 | Gly Val Asp Pro Tyr Gly Ile Glu Ala Met Ile Pro His Phe Gly Gly Leu Thr Gly Thr Asp Leu Thr Ala Met Leu Asn Asp Gin Leu Gln Pro Lys (SEQ ID Nr 14) |

### 10.3 Enzymcharakterisierung

Zur Amidasecharakterisierung wurde ein hitzebehandeltes zellfreies Extrakt eingesetzt Zellen von Klebsiella oxytoca PRS1K17 (DSM 11623) (OD₆₅₀=160) wurden durch Passieren durch die French-Presse bei 19000 psi (1309 bar) aufgebrochen. Benzonase (1 µl x 30 ml Extrakt⁻¹) wurden hinzugefugt und dann wurde das Extrakt bei 20000 x g 1 h lang zentrifugiert. Der Uberstand (ca. 20 mg x ml⁻¹ Protein) wurde für 10 min auf 70 °C erhitzt und dann wurde das prazipitierte Protein durch Zentrifugation entfernt. Der Überstand (ca. 2,0 mg x ml⁻¹) wurde auf 5,0 mg x ml⁻¹ Protein konzentriert und dann bei -20 °C aufbewahrt. Der Hitzeschritt entfernte ca 90 % unerwünschtes Protein.
Die Reaktionsrate war bis zu einer Proteinkonzentration von 0,5 mg x ml⁻¹ direkt proportional zur Proteinkonzentration Daher wurde routinemassig eine Proteinkonzentration von 0,2 mg x ml⁻¹ für die Tests eingesetzt. Fur die Bestimmung des pH-Optimums lag die Konzentration an (R,S)-2,2-HTFMPA (Substrat) bei 0,5 % (32 mM) und die Temperatur betrug 40 °C. Für den Test wurden die in Tabelle 4 aufgefuhrten Puffer eingesetzt.

**Tabelle 4**

| Puffer | pH |
|---|---|
| 100 mM MES | 6,5 |
| 100 mM HEPES | 7,0; 7,5 |
| 50 mM Phosphatpuffer | 8,0, 8,5 |
| 50/100 mM Trispuffer | 8,0, 8,5 |
| 50/100 mM Boratpuffer | 9,0,9,5 |
| 50/100 mM CAPS-Puffer | 10,0; 10,5; 11,0 |

Der Effekt der Temperatur auf die Reaktion wurde in 100 mM CAPS-Puffer (pH 10,0) bei einer Substratkonzentration von 0.5 % (32 mM) bestimmt Der Effekt auf die Substratkonzentration wurde bei 60 °C in 100 mM CAPS-Puffer (pH 10,0) und der Effekt von Methanol bei 40 und 60 °C bei einer Substratkonzentration von 1% (64 mM) in 100 mM CAPS-Puffer (pH 10,0) bestimmt Der K_{M}-Wert der Reaktion wurde mit dem Enzfitter-Programm von Biosoft ermittelt.

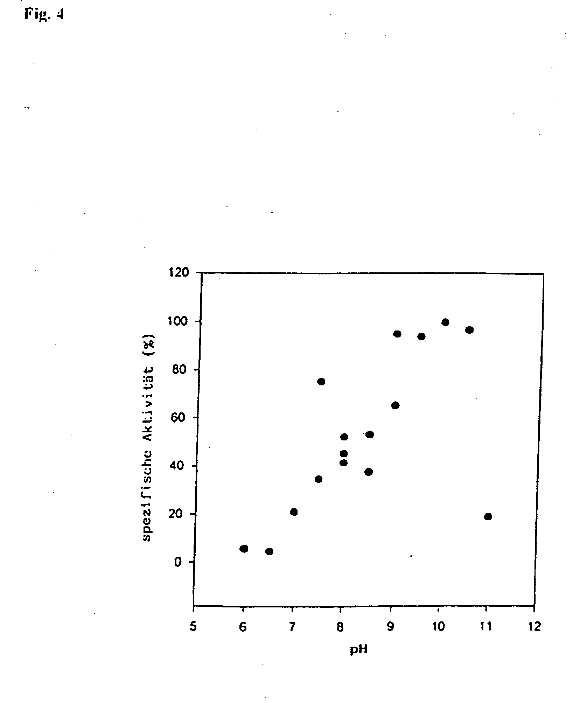

- Fig. 4: zeigt das pH-Optimum. Das pH-Optimum liegt zwischen 9.5 und 10,5 (100 mM CAPS-Puffer. Substratkonzentration 32 mM)

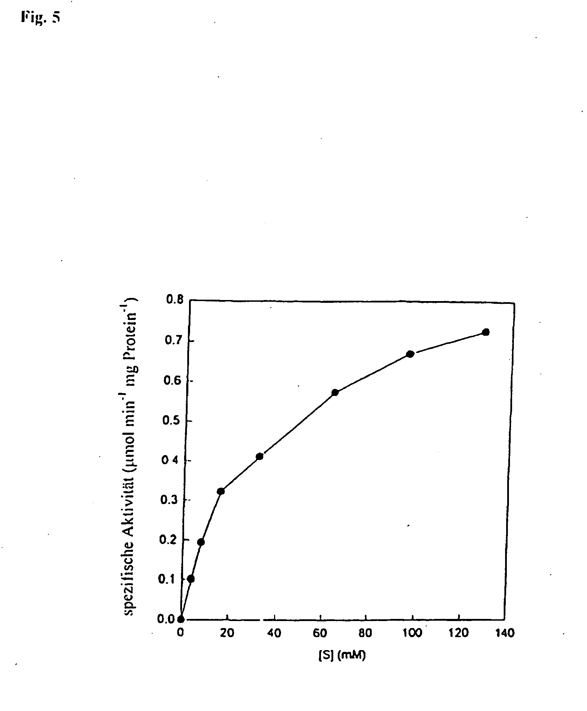

- Fig. 5: zeigt die Michaetis-Menten-Kinetik Der KM- Wert liegt bei 32 mM für (R)-2,2-HTFMPA (60 °C in 100 mM CAPS-Puffer, pH 10).

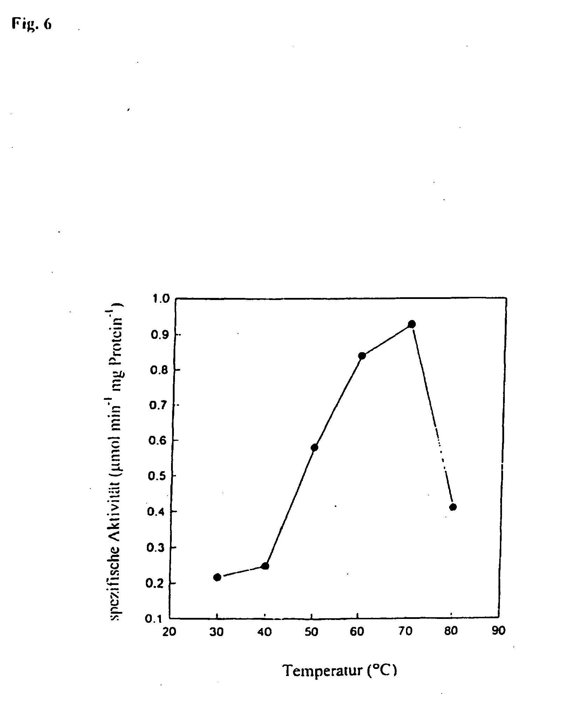

- Fig 6: zeigt das Temperaturoptimum Das Temperaturoptimum liegt bei 70 °C (100 mM CAPS-Puffer, Substratkonzentration 32 mM)

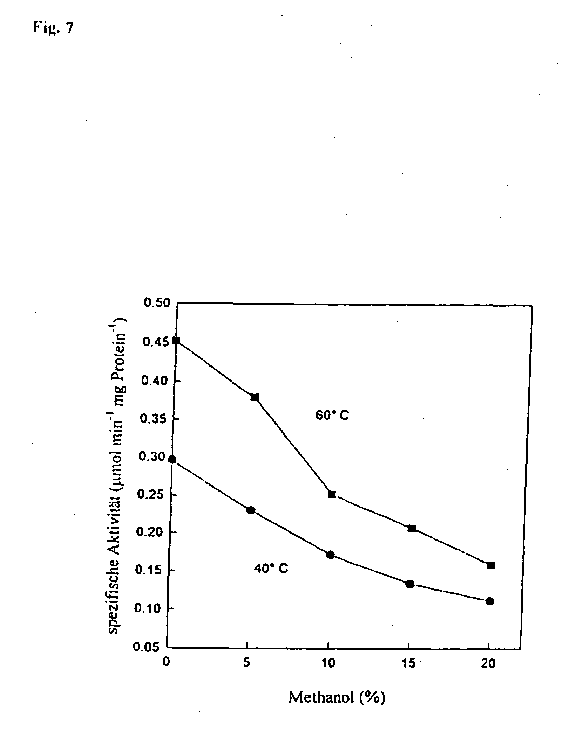

- Fig. 7: zeigt den Effekt von Methanol. Methanol-Konzentrationen zwischen 5 und 20 % inhibierten die Reaktion

### 10.4 Enzymimmobilisation

Das hitzebehandelte zellfreie Extrakt wurde mit Eupergit C (Röhm GmbH) immobilisiert Hierzu wurde Eupergit C (3,0 g) zu 15 ml hitzebehandeltern zellfreien Extrakt (Proteinkonzentration: 51 mg) in 1 M Kaliumphosphatpuffer (pH 8,0) hinzugegeben. Die Mischung wurde bei Raumtemperatur unter leichtem Ruhren 90 h lang inkubiert. Das immobilisierte Enzym wurde filtriert und 4mal mit 20 ml 100 mM Kaliumphosphatpuffer (pH 8,0) gewaschen Gebundenes Enzym am Träger (49 mg) ergab 9,5 g Feuchtgewicht an immobilisiertem Enzym, welches in 100 mM Kaliumphosphatpuffer (pH 10,0) bei 4 °C aufbewahrt wurde. Um die Aktivität und die Stabilität des immobilisierten Enzyms zu testen, wurden 5 g (25 mg Protein) in eine kleine Chromatographiesaule gefüllt. Um das Substrat (100 ml 4 %iges racemisches Amid in 100 mM CAPS-Puffer (pH 10)) zwischen Säule und Reservoir circulieren zu lassen, wurde eine peristaltische Pumpe benutzt (0,135 ml x min⁻¹). Das Ganze wurde im Wasserbad durchgefuhrt Zu gewissen Intervallen wurden fur die Analyse Proben entnommen. Das Enzym war noch nach 200 h aktiv Mit 3 Biotransformationen (jede mit 4 g racemischem Substrat, wobei die erste bei 60 °C und die anderen zwei bei 40 °C durchgeführt wurden) konnten insgesamt 6 g (S)-Amid erhalten werden. Zu Beginn der Reaktion wurden immobilisiertes Enzym (spezifische Aktivität = 47 µg x min⁻¹ x mg Protein ⁻¹) bei 60 °C hinzugefugt, was vergleichbar ist (41 %) mit nicht immobilsiertem Enzym (spezifische Aktivität. 114 µg x min⁻¹ x mg Protein⁻¹)

## Patentansprüche

1. Mikroorganismen der Gattung Klebsiella, **dadurch gekennzeichnet, dass** sie eine R-spezifische Amidohydrolase aufweisen und befähigt sind, das R-Propionsäureamid der Formel VIII 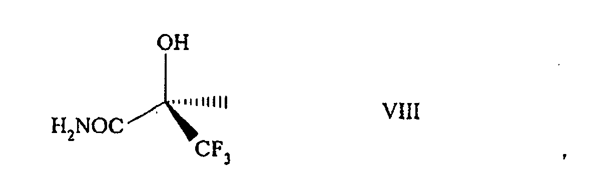 als einzige Stickstoffquelle zu verwerten, sowie zur Hydrolyse des R-Propionsäureamids der Formel VIII befähigte Enzymextrakte daraus.

2. Mikroorganismus nach Anspruch 1 der Spezies Klebsiella oxytoca, Klebsiella planticula oder Klebsiella pneumoniae.

3. Mikroorganismus nach Anspruch 2 ausgewählt aus der Gruppe bestehend aus den Stämmen K. oxytoca DSM 11009, Klebsiella planticula DSM 11354 und Klebsiella pneumoniae DSM 11355.

4. Mikroorganismus nach Anspruch 1 bis 3, **dadurch gekennzeichnet, dass** es eine Kapsel-negative Klebsiella Mutante ist oder Klebsiella oxytoca DSM11623 ist.

5. Mikroorganismen der Spezies Rhodococcus opacus DSM 11344, Arthrobacter ramosus DSM11350, Bacillus sp. DSM 11351 oder Pseudomonas sp. DSM11010, **dadurch gekennzeichnet, dass** sie eine S-spezifische Amidohydrolase aufweisen und befähigt sind, das S-Propionsäureamid der Formel VII 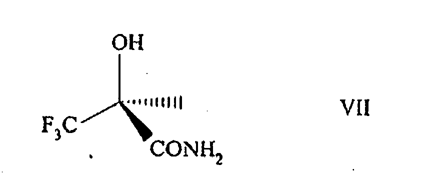 als einzige Stickstoffquelle zu verwerten, sowie zur Hydrolyse des S-Propionsäureamids der Formel VII befähigte Enzymextrakte daraus.

6. Mikroorganismen nach Anspruch 5 ausgewählt aus der Gruppe bestehend aus den Stämmen Pseudomonas sp. DSM11010, Rhodococcus opacus DSM11344, Arthrobacter ramosus DSM 11350 und Bacillus sp. DSM 11351.

7. Polypeptid, das eine R-spezifische Amidohydrolase ist und befähigt ist, (R)-3,3,3-Trifluoro-2-hydroxy-2-methylpropionsäureamid der Formel VIII 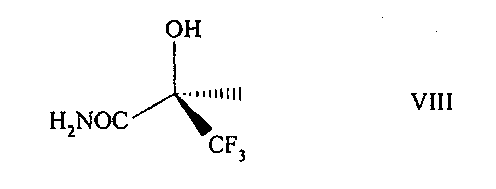 zu hydrolysieren.

8. Polypeptid nach Anspruch 7, umfassend die in Seq. ID No.2 dargestellte Aminosäuresequenz oder ein Derivat dieser Sequenz mit Aminosäuredeletionen, - substitutionen, -insertionen,-additionen und/oder -austauschen, das in seiner biologischen Funktion im wesentlichen unverändert ist.

9. DNA-Sequenz codierend für ein Polypeptid oder Polypeptiderivat nach Anspruch 7 oder 8.

10. DNA-Sequenz nach Anspruch 9, umfassend eine DNA Sequenz ausgewählt aus
a). DNA mit der in SEQ ID No. 1 dargestellten Sequenz, oder für ein Polypeptid nach Anspruch 7 codierenden Fragmente der in SEQ ID No. 1 dargestellten Sequenz, komplementären Sequenzen hierzu, sowie von diesen abgeleiteten Sequenzen, die in den codierenden Regionen aufgrund der Variation des genetischen Codes degeneriert sind; und
b.) DNA-Sequenzen, die mit den codierenden Regionen der unter (a) definierten Sequenzen unter stringenten Bedingungen bei einer Temperatur von 67°C und bei 0.8 M Salzanteil hybridisieren.

11. DNA-Sequenz nach Anspruch 9 oder 10, charakterisiert durch die Restriktionskarte gemäss Fig. 1.

12. Rekombinantes DNA-Molekül oder Vektor, enthaltend eine DNA-Sequenz nach einem der Ansprüche 9 bis 11.

13. Mikroorganismen enthaltend ein rekombinantes DNA-Molekül oder Vektor nach Anspruch 12.

14. Mikroorganismen nach Anspruch 13 ausgewählt aus Mikroorganismen der Gattung Escherichia, Pseudomonas, Comamonas, Acinetobacter, Rhizobium/Agrobacterium, Rhizobium, Bacillus, Rhodococcus oder Agrobacterium.

15. Verfahren zur Herstellung von (S)-3,3,3-Trifluoro-2-hydroxy-2-methyl-propionsäure der Formel I 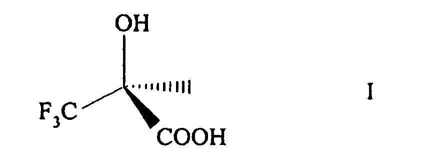 und/oder von (R)- 3,3,3-Trifluoro-2-hydroxy-2-methyl-propionsäureamid der Formel VIII 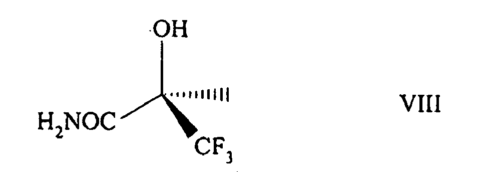 umfassend die Umsetzung des Propionsäureamids der Formel VI 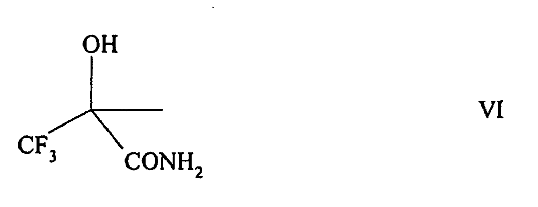 in der Form seines Racemates mittels eines Mikroorganismus gemäss den Ansprüchen 5 oder 6, bzw. den aus Ihnen isolierten Enzymen, mit stereospezifischer Amidohydrolase-Aktivität zu der Verbindung der Formel I.

16. Verfahren nach Anspruch 15, **dadurch gekennzeichnet, dass** die Verbindung der Formel I bzw. VIII isoliert wird.

17. Verfahren zur Herstellung von (R)-3,3,3-Trifluoro-2-hydroxy-2-methyl-propionsäure der Formel II 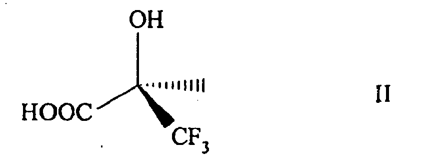 und/oder von (S)- 3,3,3-Trifluoro-2-hydroxy-2-methyl-propionsäureamid der Formel VII 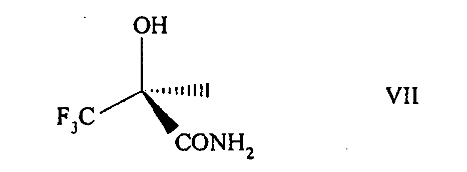 umfassend die Umsetzung des Propionsäureamids der Formel VI 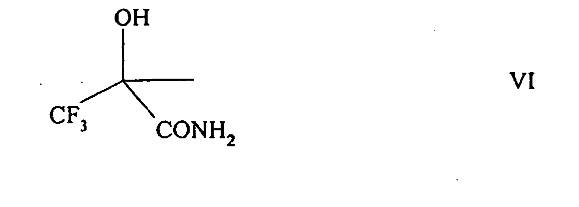 in der Form seines Racemates mittels eines Mikroorganismus oder Enzymextrakten daraus gemäss den Ansprüchen 1 bis 4 bzw. den aus Ihnen isolierten Enzymen mit stereospezifischer Amidohydrolase-Aktivität zu der Verbindung der Formel II.

18. Verfahren nach Anspruch 15, **dadurch gekennzeichnet, dass** die Verbindung der Formel II isoliert wird und/oder die bei dieser Umsetzung anfallenden Verbindung der Formel VII.

19. Verfahren nach Anspruch 17 oder 18, **dadurch gekennzeichnet, dass** das isolierte Enzym ein Polypeptid oder Polypeptidderivat gemäss den Ansprüchen 7 oder 8 ist.

20. Verfahren zur Herstellung von (S)- 3,3,3-Trifluoro-2-hydroxy-2-methyl-propionsäure, **dadurch gekennzeichnet, dass** man als Zwischenprodukt (S)- 3,3,3-Trifluoro-2-hydroxy-2-methyl-propionsäureamid der Formel VII nach dem Verfahren der Ansprüche 17, 18 oder 19 herstellt und es dann chemisch in Gegenwart einer Base oder mikrobiologisch mittels Mikroorganismen der Spezies Rhodococcus equi oder Rhodococcus rhodochrous zur Verbindung der Formel I 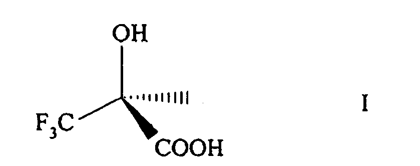 hydrolysiert.

21. Verfahren zur Herstellung von (R)-3,3,3-Trifluoro-2-hydroxy-2-methyl-propionsäure, **dadurch gekennzeichnet, dass** man als Zwischenprodukt (R)- 3,3,3-Trifluoro-2-hydroxy-2-methyl-propionsäureamid der Formel VIII nach dem Verfahren der Ansprüche 15 oder 16 herstellt und es chemisch in Gegenwart einer Base oder mikrobiologisch mittels Mikroorganismen der Spezies Rhodococcus equi oder Rhodococcus rhodochrous zur Verbindung der Formel II 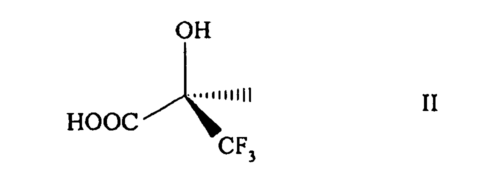 hydrolysiert.

22. Verfahren nach Anspruch 20 oder 21, **dadurch gekennzeichnet, dass** chemisch in Gegenwart einer Base hydrolysiert wird.

23. Verfahren nach einem der Ansprüche 20 bis 22, **dadurch gekennzeichnet, dass** die Base ein Alkalimetallhydroxid ist.

24. Verfahren nach einem der Ansprüche 15 bis 21, **dadurch gekennzeichnet, dass** das Propionsäureamid der Formel VI **dadurch** hergestellt wird, dass in der ersten Stufe Trifluoroacetessigester der Formel III 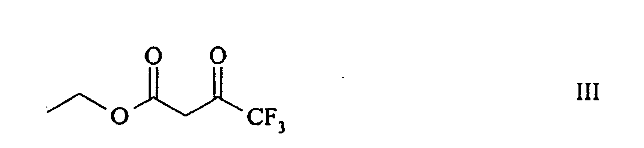 mit einer Mineralsäure in Trifluoraceton der Formel IV 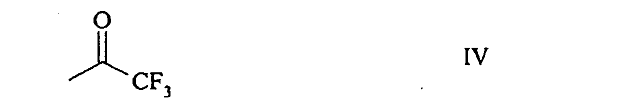 überführt wird, dieses in der zweiten Stufe mit einem Cyanid in das Propionsäurenitril der Formel V 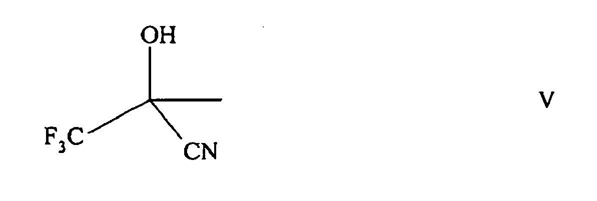 überführt wird und dieses in der dritten Stufe entweder chemisch mit einer konzentrierten Mineralsäure oder mikrobiologisch mit einem Mikroorganismus der Gattung Rhodococcus, der keine Amidase enthält und somit nicht befähigt ist ein Amid in die entsprechende Säure zu überführen, in das Propionsäureamid der Formel VI überführt wird.

25. Verfahren nach Anspruch 24, **dadurch gekennzeichnet, dass** der Mikroorganismus ein mutierter Mikroorganismus der Spezies Rhodococcus equi oder Rhodococcus rhodochrous ist, der keine Amidase enthält.

26. Verfahren nach Anspruch 25, **dadurch gekennzeichnet, dass** der Mikroorganismus Rhodococcus equi ,TG328-2' DSM 11636 ist.

27. Verfahren nach Anspruch 24, **dadurch gekennzeichnet, dass** man in der ersten und/oder dritten Stufe als Mineralsäure Schwefelsäure, Phosphorsäure oder Salpetersäure verwendet.

28. Verfahren nach Anspruch 24, **dadurch gekennzeichnet, dass** man in der zweiten Stufe als Cyanid ein Alkalimetallcyanid verwendet.

29. (R)- 3,3,3-Trifluoro-2-hydroxy-2-methyl-propionsäureamid.

30. (S)- 3,3,3-Trifluoro-2-hydroxy-2-methyl-propionsäureamid.

31. Polypetid, das eine S-spezifische Amidohydrolase ist und befähigt ist, (S)- 3,3,3-Trifluoro-2-hydroxy-2-methylpropionsäureamid der Formel VII 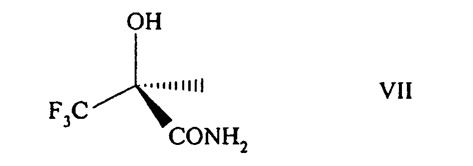 zu hydrolysieren, erhältlich aus den Mikroorganismen der Spezies Pseudomonas sp. DSM 11010.

## Claims

1. Microorganisms of the genus Klebsiella, **characterized in that** they have an R-specific amidohydrolase and are capable of utilizing the R-propionamide of the formula VIII 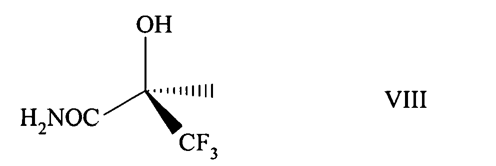 as sole nitrogen source, and enzyme extracts therefrom which are capable of hydrolysing the R-propionamide of the formula VIII.

2. Microorganism according to Claim 1 of the species Klebsiella oxytoca, Klebsiella planticula or Klebsiella pneumoniae.

3. Microorganism according to Claim 2 selected from the group consisting of the strains K. oxytoca DSM 11009, Klebsiella planticula DSM 11354 and Klebsiella pneumoniae DSM 11355.

4. Microorganism according to Claim 1 to 3, **characterized in that** it is a capsule-negative Klebsiella mutant or is Klebsiella oxytoca DSM11623.

5. Microorganisms of the species Rhodococcus opacus DSM11344, Arthrobacter ramosus DSM11350, Bacillus sp. DSM 11351 or Pseudomonas sp. DSM11010, **characterized in that** they have an S-specific amidohydrolase and are capable of utilizing the S-propionamide of the formula VII 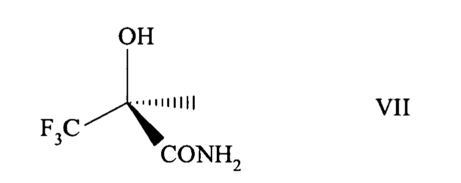 as sole nitrogen source, and enzyme extracts thereof which are capable of hydrolysing the S-propionamide of the formula VII.

6. Microorganisms according to Claim 5 selected from the group consisting of the strains Pseudomonas sp. DSM11010, Rhodococcus opacus DSM11344, Arthrobacter ramosus DSM 11350 and Bacillus sp. DSM 11351.

7. Polypeptide which is an R-specific amidohydrolase and is capable of hydrolysing (R)-3,3,3-trifluoro-2-hydroxy-2-methylpropionamide of the formula VIII

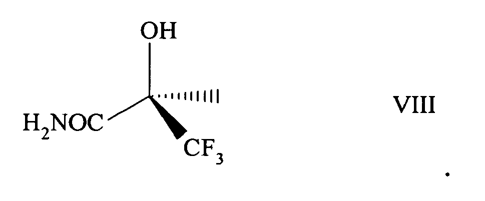

8. Polypeptide according to Claim 7, comprising the amino acid sequence depicted in Seq. ID No. 2 or a derivative of this sequence with amino acid deletions, substitutions, insertions, additions and/or exchanges, which is essentially unmodified in its biological function.

9. DNA sequence coding for a polypeptide or polypeptide derivative according to Claim 7 or 8.

10. DNA sequence according to Claim 9, comprising a DNA sequence selected from
a). DNA with the sequence shown in SEQ ID No. 1, or fragments, coding for a polypeptide according to Claim 7, of the sequence shown in SEQ ID No. 1, sequences which are complementary thereto, and sequences which are derived from them and which are degenerate in the encoding regions due to the variation of the genetic code; and
b). DNA sequences which hybridize with the encoding regions of the sequences defined under (a) under stringent conditions at a temperature of 67°C and a salt content of 0.8 M.

11. DNA sequence according to Claim 9 or 10, **characterized by** the restriction map shown in Fig. 1.

12. Recombinant DNA molecule or vector comprising a DNA sequence according to any of Claims 9 to 11.

13. Microorganisms comprising a recombinant DNA molecule or vector according to Claim 12.

14. Microorganisms according to Claim 13 selected from microorganisms of the genus Escherichia, Pseudomonas, Comamonas, Acinetobacter, Rhizobium/Agrobacterium, Rhizobium, Bacillus, Rhodococcus or Agrobacterium.

15. Process for the preparation of (S)-3,3,3-tri-fluoro-2-hydroxy-2-methylpropionic acid of the formula I 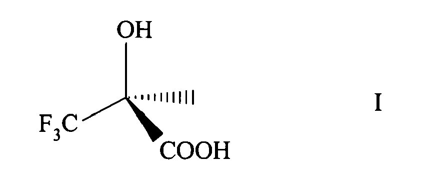 and/or of (R)-3,3,3-trifluoro-2-hydroxy-2-methyl-propionamide of the formula VIII 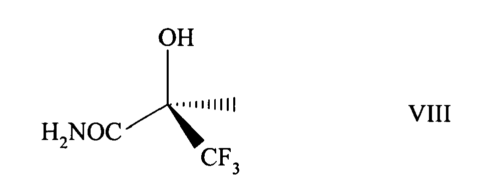 comprising the conversion of the propionamide of the formula VI 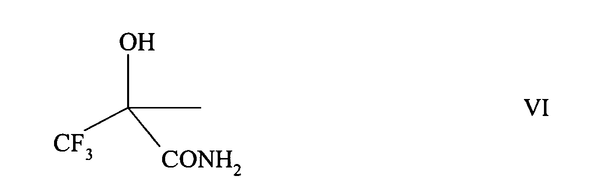 in the form of its racemate by means of a microorganism according to Claims 5 or 6, or the enzymes isolated therefrom and having stereospecific amidohydrolase activity, to the compound of the formula I.

16. Process according to Claim 15, **characterized in that** the compound of the formula I or VIII is isolated.

17. Process for the preparation of (R)-3,3,3-tri-fluoro-2-hydroxy-2-methylpropionic acid of the formula II 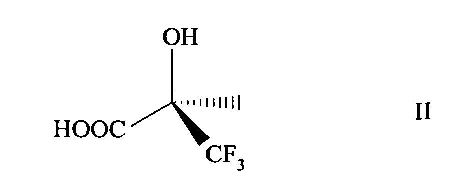 and/or of (S)-3,3,3-trifluoro-2-hydroxy-2-methyl-propionamide of the formula VII 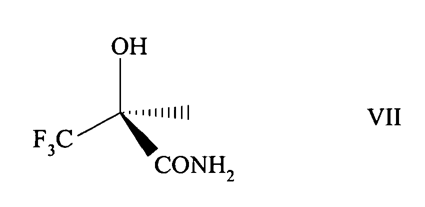 comprising the conversion of the propionamide of the formula VI 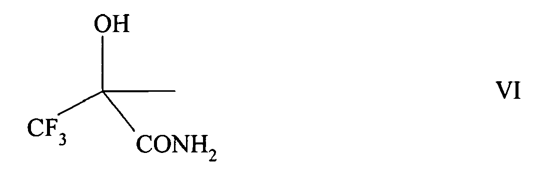 in the form of its racemate by means of a microorganism or enzyme extracts therefrom according to Claims 1 to 4, or the enzymes isolated therefrom and having stereospecific amidohydrolase activity, to the compound of the formula II.

18. Process according to Claim 15, **characterized in that** the compound of the formula II is isolated and/or the compound of the formula VII resulting in this conversion.

19. Process according to Claim 17 or 18, **characterized in that** the isolated enzyme is a polypeptide or polypeptide derivative according to Claims 7 or 8.

20. Process for the preparation of (S)-3,3,3-tri-fluoro-2-hydroxy-2-methylpropionic acid, **characterized in that** (S)-3,3,3-trifluoro-2-hydroxy-2-methylpropionamide of the formula VII is prepared as intermediate by the process of Claims 17, 18 or 19, and it is then hydrolysed chemically in the presence of a base or microbiologically by means of microorganisms of the species Rhodococcus equi or Rhodococcus rhodochrous, to the compound of the formula I

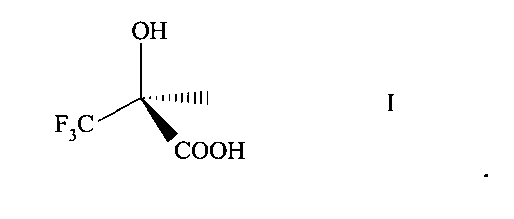

21. Process for the preparation of (R)-3,3,3-tri-fluoro-2-hydroxy-2-methylpropionic acid, **characterized in that** (R)-3,3,3-trifluoro-2-hydroxy-2-methylpropionamide of the formula VIII is prepared as intermediate by the process of Claims 15 or 16, and it is hydrolysed chemically in the presence of a base or microbiologically by means of microorganisms of the species Rhodococcus equi or Rhodococcus rhodochrous, to the compound of the formula II

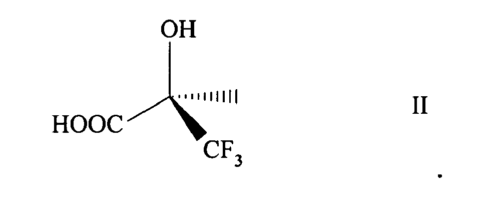

22. Process according to Claim 20 or 21, **characterized in that** hydrolysis is carried out chemically in the presence of a base.

23. Process according to any of Claims 20 to 22, **characterized in that** the base is an alkali metal hydroxide.

24. Process according to any of Claims 15 to 21, **characterized in that** the propionamide of the formula VI is prepared by converting, in the first step, trifluoroacetate of the formula III 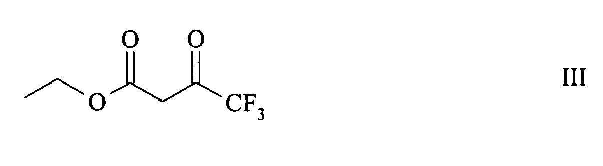 into trifluoroacetone of the formula IV 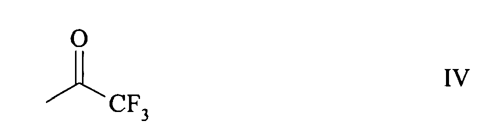 using a mineral acid, converting the former, in the second step, into the propionitrile of the formula V 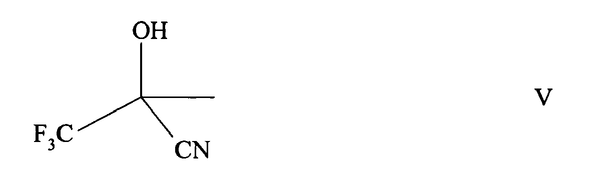 using a cyanide, and converting the former, in the third step, into the propionamide of the formula VI either chemically using a concentrated mineral acid or microbiologically using a microorganism of the genus Rhodococcus which comprises no amidase and thus is incapable of converting an amide into the corresponding acid.

25. Process according to Claim 24, **characterized in that** the microorganism is a mutated microorganism of the species Rhodococcus equi or Rhodococcus rhodochrous which comprises no amidase.

26. Process according to Claim 25, **characterized in that** the microorganism is Rhodococcus equi 'TG328-2' DSM 11636.

27. Process according to Claim 24, **characterized in that** sulphuric acid, phosphoric acid or nitric acid is used as mineral acid in the first and/or third step.

28. Process according to Claim 24, **characterized in that** an alkali metal cyanide is used as cyanide in the second step.

29. (R)-3,3,3-Trifluoro-2-hydroxy-2-methyl-propionamide.

30. (S)-3,3,3-Trifluoro-2-hydroxy-2-methylpropion-amide.

31. Polypeptide which is an S-specific amidohydrolase and is capable of hydrolysing (S)-3,3,3-trifluoro-2-hydroxy-2-methylpropionamide of the formula VII 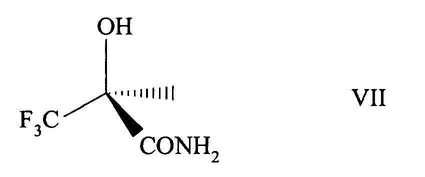 obtainable from the microorganisms of the species Pseudomonas sp. DSM 11010.

## Revendications

1. Microorganismes du genre Klebsiella, **caractérisés en ce qu'**ils possèdent une amidohydrolase R-spécifique, et sont capables d'utiliser le R-propionamide de formule VIII 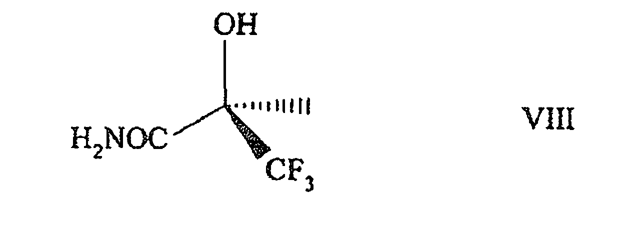 en tant que source d'azote unique, ainsi que leurs extraits enzymatiques aptes à l'hydrolyse du R-propionamide de formule VIII.

2. Microorganisme selon la revendication 1 de l'espèce Klebsiella oxytoca, Klebsiella planticula ou Klebsiella pneumoniae.

3. Microorganisme selon la revendication 2, choisi dans le groupe consistant en les souches K. oxytoca DSM 11009, Klebsiella planticula DSM 11354 et Klebsiella pneumoniae DSM 11355.

4. Microorganisme selon les revendications 1 à 3, **caractérisé en ce qu'**il s'agit d'un mutant de Klebsiella capsule-négatif ou de Klebsiella oxytoca DSM 11623.

5. Microorganismes de l'espèce Rhodococcus opacus DSM 11344, Arthrobacter ramosus DSM 11350, Bacillus sp. DSM 11351 ou Pseudomonas sp. DSM 11010, **caractérisés en ce qu'**ils comprennent une amidohydrolase S-spécifique, et sont aptes à utiliser le S-propionamide de formule VII 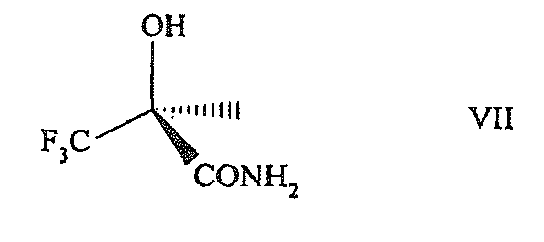 en tant que source d'azote unique, ainsi que leurs extraits enzymatiques aptes à l'hydrolyse du S-propionamide de formule VII.

6. Microorganismes selon la revendication 5, choisis dans le groupe consistant en les souches Pseudomonas sp. DSM11010, Rhodococcus opacus DSM 11344, Arthrobacter ramosus DSM 11350 et Bacillus sp. DSM 11351.

7. Polypeptide qui est une amidohydrolase R-spécifique, et est apte à l'hydrolyse du (R)-3,3,3-trifluoro-2-hydroxy-2-méthylpropionamide de formule VIII.

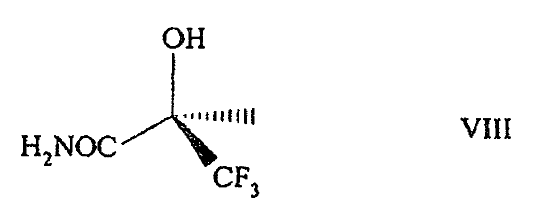

8. Polypeptide selon la revendication 7, comprenant la séquence d'acides aminés représentée dans SEQ ID N° 2 ou un dérivé de cette séquence avec des délétions, des substitutions, des insertions, des additions et/ou des échanges d'acides aminés, dont la fonction biologique est pour l'essentiel non modifiée.

9. Séquence d'ADN codant pour un polypeptide ou un dérivé de polypeptide selon la revendication 7 ou 8.

10. Séquence d'ADN selon la revendication 9, comprenant une séquence d'ADN choisie parmi
a) un ADN ayant la séquence représentée dans SEQ ID N° 1, ou des fragments codant pour un polypeptide selon la revendication 7 de la séquence représentée dans SEQ ID N° 1, leurs séquences complémentaires, ainsi que les séquences qui en dérivent, qui dans les régions codantes sont dégénérées en raison de la variation du code génétique ; et
b) des séquences d'ADN qui s'hybrident aux régions codantes des séquences définies en a), dans des conditions stringentes à une température de 67°C, et pour une concentration de sels de 0,8 M.

11. Séquence d'ADN selon la revendication 9 ou 10, **caractérisée par** la carte de restriction selon la Figure 1.

12. Molécule d'ADN recombinant ou vecteur contenant une séquence d'ADN selon l'une des revendications 9 à 11.

13. Microorganismes contenant une molécule d'ADN recombinant ou un vecteur selon la revendication 12.

14. Microorganismes selon la revendication 13, choisis parmi les microorganismes des genres Escherichia, Pseudomonas, Comamonas, Acinetobacter, Rhizobium/Agrobacterium, Rhizobium, Bacillus, Rhodococcus ou Agrobacterium.

15. Procédé de préparation d'acide (S)-3,3,3-trifluoro-2-hydroxy-2-méthyl-propionique de formule I 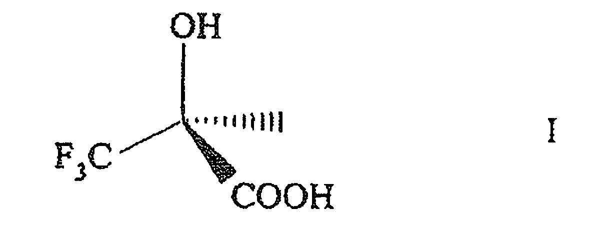 et/ou de (R)-3,3,3-trifluoro-2-hydroxy-2-méthyl-propionamide de formule VIII 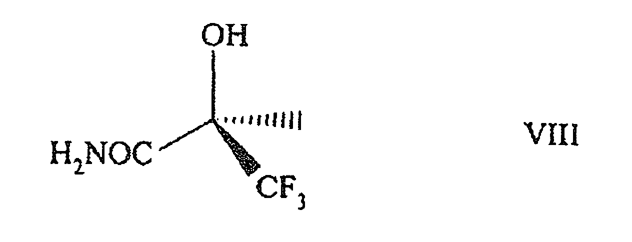 comprenant la réaction du propionamide de formule VI 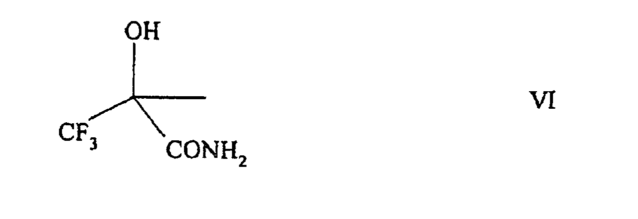 sous forme de son racémate, à l'aide d'un microorganisme selon les revendications 5 ou 6, ou des enzymes isolées à partir de lui, et ayant une activité d'amidohydrolase stéréospécifique, pour donner le composé de formule I.

16. Procédé selon la revendication 15, **caractérisé en ce que** c'est le composé de formule I ou VIII qui est isolé.

17. Procédé de préparation de l'acide (R)-3,3,3-trifluoro-2-hydroxy-2-méthyl-propionique de formule II 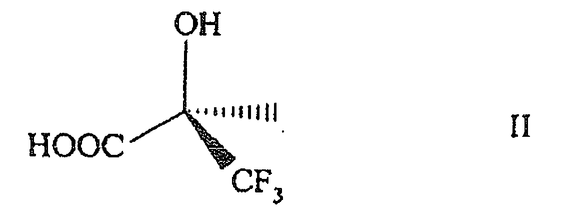 et/ou du (S)-3,3,3-trifluoro-2-hydroxy-2-méthyl-propionamide de formule VII 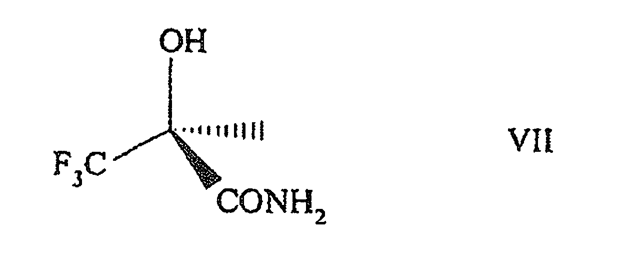 comprenant la réaction du propionamide de formule VI 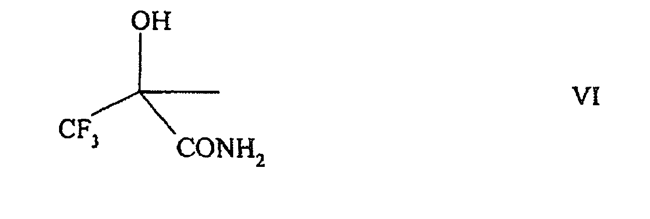 sous forme de son racémate, à l'aide d'un microorganisme ou d'extraits enzymatiques de ce dernier selon les revendications 1 à 4, ou des enzymes isolées à partir de lui, ayant une activité d'amidohydrolase stéréospécifique, pour donner le composé de formule II.

18. Procédé selon la revendication 15, **caractérisé en ce que** c'est le composé de formule II et/ou le composé de formule VII obtenu lors de cette réaction, qui sont isolés.

19. Procédé selon la revendication 17 ou 18, **caractérisé en ce que** l'enzyme isolée est un polypeptide ou un dérivé de polypeptide selon les revendications 7 ou 8.

20. Procédé de préparation d'acide (S)-3,3,3-trifluoro-2-hydroxy-2-méthyl-propionique, **caractérisé en ce qu'**on prépare en tant que produit intermédiaire le (S)-3,3,3-trifluoro-2-hydroxy-2-méthyl-propionamide de formule VII par le procédé des revendications 17, 18 ou 19, puis qu'on l'hydrolyse par voie chimique en présence d'une base ou par voie microbiologique à l'aide de microorganismes des espèces Rhodococcus equi ou Rhodococcus rhodochrous, pour donner le composé de formule I

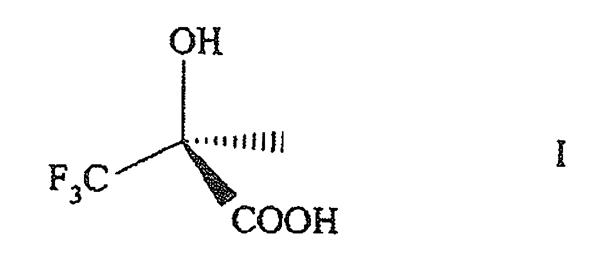

21. Procédé de préparation de l'acide (R)-3,3,3-trifluoro-2-hydroxy-2-méthyl-propionique, **caractérisé en ce qu'**on prépare en tant que produit intermédiaire le (R)-3,3,3-trifluoro-2-hydroxy-2-méthyl-propionamide de formule VIII par le procédé des revendications 15 ou 16, et qu'on l'hydrolyse par voie chimique en présence d'une base ou par voie microbiologique à l'aide de microorganismes des espèces Rhodococcus equi ou Rhodococcus rhodochrous, pour donner le composé de formule II

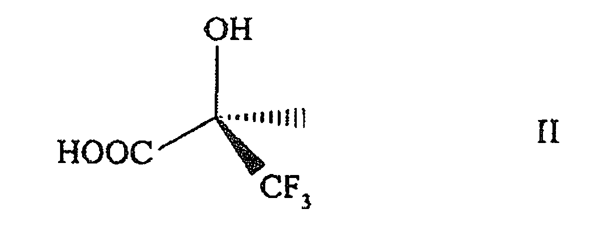

22. Procédé selon la revendication 20 ou 21, **caractérisé en ce que** l'hydrolyse a lieu par voie chimique en présence d'une base.

23. Procédé selon l'une des revendications 20 à 22, **caractérisé en ce que** la base est un hydroxyde d'un métal alcalin.

24. Procédé selon l'une des revendications 15 à 21, **caractérisé en ce qu'**on prépare le propionamide de formule VI en convertissant dans une première étape le trifluoracétoacétate de formule III 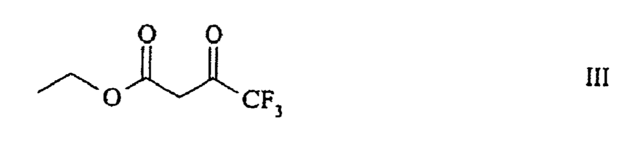 avec un acide minéral en la trifluoracétone de formule IV 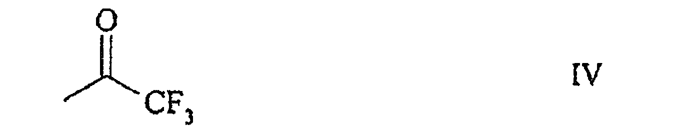 on convertit cette dernière dans une deuxième étape avec un cyanure en le propiononitrile de formule V 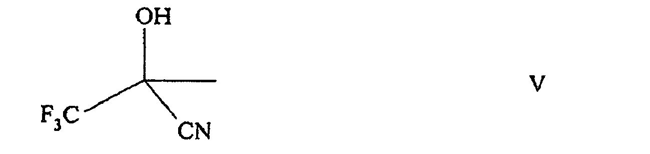 et, dans une troisième étape, on convertit ce dernier soit par voie chimique avec un acide minéral concentré, soit par voie microbiologique avec un microorganisme du genre Rhodococcus qui ne contient pas d'amidase et ainsi n'est pas apte à convertir l'amide en l'acide correspondant, en le propionamide de formule VI.

25. Procédé selon la revendication 24, **caractérisé en ce que** le microorganisme est un microorganisme muté de l'espèce Rhodococcus equi ou Rhodococcus rhodochrous, qui ne contient pas d'amidase.

26. Procédé selon la revendication 25, **caractérisé en ce que** le microorganisme est Rhodococcus equi, TG328-2' DSM 11636.

27. Procédé selon la revendication 24, **caractérisé en ce qu'**on utilise dans la première et/ou dans la troisième étapes, en tant qu'acide minéral, de l'acide sulfurique, de l'acide phosphorique ou de l'acide nitrique.

28. Procédé selon la revendication 24, **caractérisé en ce qu'**on utilise en tant que cyanure dans la deuxième étape un cyanure d'un métal alcalin.

29. (R)-3,3,3-trifluoro-2-hydroxy-2-méthyl-propionamide.

30. (S)-3,3,3-trifluoro-2-hydroxy-2-méthyl-propionamide.

31. Polypeptide qui est une amidohydrolase S-spécifique et est apte à hydrolyser le (S)-3,3,3-trifluoro-2-hydroxy-2-méthyl-propionamide de formule VII 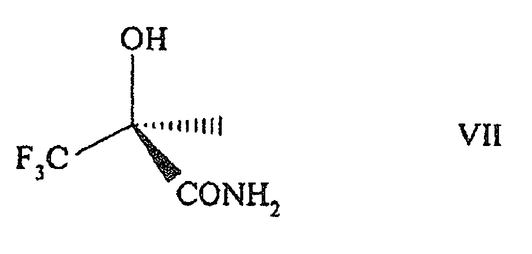 pouvant être obtenu à partir des microorganismes de l'espèce Pseudomonas sp. DSM 11010.
